# EUROPÄISCHE PATENTSCHRIFT

(11) **EP 3 736 590 B1**
(45) Veröffentlichungstag und Bekanntmachung des Hinweises auf die Patenterteilung: **17.01.2024**
(21) Anmeldenummer: 19173532.3
(22) Anmeldetag: 09.05.2019
(51) Int. Cl.: G01R 33/38

(54) **MAGNETRESONANZTOMOGRAPHIESYSTEM ZUR SIMULTANEN MESSUNG MEHRERER PATIENTEN**
MRI SYSTEM FOR SIMULTANEOUS IMAGING OF MULTIPLE PATIENTS
SYSTÈME D'IMAGERIE PAR RÉSONANCE MAGNÉTIQUE D'IMAGERIE SIMULTANÉE DE MULTIPLES PATIENTS

(43) Veröffentlichungstag der Anmeldung: 11.11.2020
(73) Patentinhaber: Siemens Healthcare GmbH, 91052 Erlangen (DE)
(72) Erfinder: Popescu, Stefan, 91056 Erlangen (DE)
(74) Vertreter: Siemens Healthineers Patent Attorneys

(56) Entgegenhaltungen:
- EP-A2- 0 311 294
- DE-A1- 19 610 266
- JP-A- H10 234 704
- JP-A- S61 159 950
- US-A- 5 614 880
- US-B2- 7 355 499
- EPSTEIN C L ET AL: "A novel technique for imaging with inhomogeneous fields", JOURNAL OF MAGNETIC RESONANCE, ACADEMIC PRESS, ORLANDO, FL, US, Bd. 183, Nr. 2, 1. Dezember 2006 (2006-12-01), Seiten 183-192, XP024919389, ISSN: 1090-7807, DOI: 10.1016/J.JMR.2006.08.012 [gefunden am 2006-12-01]

## Beschreibung

Die Erfindung betrifft ein Magnetresonanztomographie-System ("MR-System") und ein Verfahren zur Messung von Rohdaten für eine Magnetresonanztomographieaufnahme.

Ganzkörper-MR-Scanner gehören heute mit Kosten von z.T. mehreren Millionen Euro zu den teuersten Geräten für die medizinische Bildgebung. Diese Scanner verwenden in der Regel einen starken supraleitenden Magneten, der durchaus ein Gewicht von mehreren Tonnen haben kann. Die Herstellungs- und Betriebskosten solcher Magnete, betragen oft zwischen 70% und 80% der gesamten Systemkosten.

Das von dem Gerät erzeugte Grundmagnetfeld ist so stark, dass es auch massive Metallobjekte wie z.B. einen Patientenstuhl im Untersuchungsraum stark beschleunigen kann. In den frühen achtziger Jahren waren MR-Scanner im Allgemeinen nicht abgeschirmt, und die Magneträume wurden extrem groß dimensioniert (2 bis 4-mal so groß wie die Bildgebungsräume), um die großen Streumagnetfelder zu erfassen. Die Zone dieser Streumagnetfelder ist der sogenannte "kontrollierte Bereich", in dem die Intensität des statischen Magnetfelds größer als 0,5 G bzw. 50 µT ist.

Zur Reduktion des Streufeldes enthalten die modernen aktiv geschirmten Grundfeldmagnetkonstruktionen mindestens einen zusätzlichen Satz von Magnetspulen, die dem von den Hauptspulen erzeugten äußeren Feld entgegenwirken. Beispielsweise könnten die Hauptwicklungen ein Grundmagnetfeld von 2,0 T erzeugen, während die Abschirmspulen ein Feld von 0,5 T in die entgegengesetzte Richtung erzeugen. Der Nettoeffekt ist ein Grundmagnetfeld von 1,5 T in der Mitte des Grundfeldmagneten.

Obwohl die Abschirmung die nutzbare Feldstärke im Betriebsvolumen verringert, ist der Reduktionseffekt auf das Streufeld wesentlich größer. Dies reduziert jedoch wesentlich die Effizienz des Grundfeldmagneten, was einen höheren Strom durch die Magnetspulen und/oder eine höhere Anzahl von Wicklungen pro Spule erfordert. Beides erhöht die Kosten.

Um diese Kosten niedrig zu halten, müssen die Hersteller die Länge des Grundfeldmagneten vergrößern und die Öffnung des Patiententunnels (engl.: "bore") begrenzen. Damit schließen die Ganzkörper-MR-Scanner den Patienten jedoch auf engstem Raum ein. Dies ist ein Hauptproblem für Patienten mit Klaustrophobie, die den MR-Nutzen beeinflussen und begrenzen. Klinische Studien haben gezeigt, dass bis zu 15% aller MR-Patienten an Angstzuständen basierend auf Klaustrophobie leiden, und entweder mit nicht untersucht werden können oder eine Sedierung benötigen. Bei weltweit jährlich 80 Millionen MR-Verfahren werden aufgrund von Klaustrophobie rund 2 Millionen MR-Untersuchungen nicht abgeschlossen. In finanzieller Hinsicht entspricht dies bei einem angenommenen Wert von 500 € pro Verfahren einem Verlust von 1 Milliarde Euro.

Ein weiterer Nachteil ist, dass der Zugang des medizinischen Personals zum Patienten während der Bildgebung aufgrund des Magnetfeldes und der Enge sehr eingeschränkt oder nicht möglich ist. Dies hat bisher dazu geführt, dass die interventionelle MRI bisher keine signifikante klinische Anwendung hat.

Aus dem Stand der Technik sind zahlreiche Versuche bekannt, die eine Vielzahl von Konstruktionen mit niedrigem Feld und offenem Magneten darstellen, mit dem Ziel, die Magnetkosten zu senken, den Zugang zum Patienten zu verbessern oder klaustrophobische Effekte zu vermeiden. Im Allgemeinen verwenden diese Scanner entweder Permanentmagnete oder bei Raumtemperatur betriebene Elektromagnete. Die statische Magnetfeldstärke liegt bei weit unter 0,5 T.

Oftmals ist das Abbildungsvolumen von alternativen Scannern stark begrenzt, da die Magnetpole sehr nahe am Körper des Patienten liegen, was mit den Zielen eines offenen Zugangs oder eines weitgehend uneingeschränkten Patientenraums in Konflikt steht. Beispielsweise offenbart die US 2004/0066194 A1 eine unilaterale oder in Patiententischplatte befindliche MR-Scannerarchitektur. Aufgrund des kleinen Elektromagneten nimmt die Magnetfeldintensität des statischen Grundmagnetfelds B0 hier jedoch im Abstand vom Grundfeldmagneten stark ab. Diese beträgt von 0,5 T an der Magnetoberfläche bis zu 0,05 T bei 30 cm (ein Faktor x10 über einen Spalt von 30 cm). Darüber hinaus werden für solche Magnete sperrige Elektromagnete mit einem sperrigen Eisenjoch benötigt, die den Zugang zum Patienten erheblich einschränken. Darüber hinaus ist das Magnetfeld in allen Raumrichtungen stark nichtlinear und anisotrop. Ein weiterer Nachteil besteht darin, dass das Abbildungsvolumen auf z. 30 x 30 x 20 cm begrenzt ist.

Nicht supraleitende Elektromagnete werden zudem während des Betriebs heiß und benötigen eine effektive Kühlung. Um eine Ionenkontamination zu vermeiden, wird dies oft mittels Hohlleitern und Wasseraufbereitungssystemen realisiert, welche ebenfalls eine Offenheit eines Scanners einschränken.

Es ist eine Aufgabe der vorliegenden Erfindung, ein alternatives Magnetresonanztomographie-System anzugeben, mit dem die oben beschriebenen Nachteile vermieden oder zumindest reduziert werden.

Diese Aufgabe wird durch ein Magnetresonanztomographie-System gemäß Patentanspruch 1 und ein Verfahren gemäß Patentanspruch 10 gelöst.

Das erfindungsgemäße Magnetresonanztomographie-System umfasst eine Grundfeldmagnetanordnung (welche auch als "Grundfeldmagnet-System" bezeichnet werden kann) zur Erzeugung eines Grundmagnetfelds und mehrere räumlich getrennte Messplätze. Die Grundfeldmagnetanordnung umfasst diejenigen Grundfeldmagneten, die das bestimmungsgemäße Grundmagnetfeld erzeugen. Dies kann z.B. ein bekannter Grundfeldmagnet in Form eines Solenoides sein. Die Messplätze sind zur Durchführung von MR-Messungen ausgelegt, umfassen also (zumindest temporär wie später noch erläutert wird) die dafür notwendige Infrastruktur, wie z. B. die erwähnten Messeinrichtungen. Diesbezüglich können die Messplätze auch als "Magnetresonanz-Messplätze" (MR-Messplätze) bezeichnet werden, um diesen Umstand zu unterstreichen.

Wichtig für die Erfindung ist, dass das Magnetresonanztomographie-System dazu ausgelegt ist, das bestimmungsgemäße Grundmagnetfeld (bewusst bzw. beabsichtigt) gemeinsam für die Messplätze zu nutzen. Es stehen also nicht einfach zwei getrennte MR-Systeme zur Verfügung, sondern es wird ein und dasselbe Grundmagnetfeld verwendet. Zwei unterschiedliche Magnetfelder, z. B. von zwei an sich völlig getrennten MR-Systemen mit eigenständigen Grundfeldmagnetsystemen, deren (wie oben erwähnt in der Regel durch eine geeignete Abschirmung stark reduzierte) Streufelder unbeabsichtigt bzw. zufällig möglicherweise miteinander interferieren, sind nicht Teil der Erfindung. Bevorzugt kann beim Grundfeldmagnet auf eine Abschirmung verzichtet werden bzw. kann dessen Abschirmung zumindest schwächer als normal sein, um das Grundmagnetfeld z. B. erst außerhalb eines Aktionsbereichs, in welchem die Messplätze angeordnet sind, abzuschirmen.

Die Erfindung ermöglicht also, wie später noch genauer erläutert wird, parallel zu einer Durchführung einer Magnetresonanztomographieaufnahme bzw. MR-Untersuchung die Durchführung von zusätzlichen Untersuchungen. Dies bedeutet, dass zum einen mehr Patienten untersucht werden können und eine Amortisierung der Kosten für ein MR-System schneller eintritt. Zum anderen bietet dies die Möglichkeit, auch in nicht so finanzstarken Gebieten die Anzahl der zur Verfügung stehenden MR-Messplätze zu erhöhen. Auch kann der Ressourcenaufwand für jeden MR-Messplatz verringert werden.

Ein bevorzugtes Magnetresonanztomographie-System umfasst hierzu also mindestens zwei Messeinrichtungen, die zumindest zum Empfang von MR-Signalen, also zur Akquisition von Rohdaten, ausgelegt sind. Bevorzugt sind die Messeinrichtungen auch zur Applikation einer Pulssequenz zur Anregung ausgelegt, um die MR-Signale zu induzieren. Dabei ist jede der Messeinrichtungen zur Durchführung einer Messung im Rahmen einer Magnetresonanztomographieaufnahme an einem der Messplätze ausgelegt, die vorzugsweise voneinander unabhängig sind und gegebenenfalls auch gleichzeitig erfolgen können, wie unten noch weiter erläutert wird.

Eine Messeinrichtung zur Messung mit einem erfindungsgemäßen Magnetresonanztomographie-System an einem Messplatz umfasst zumindest ein HF-Sendesystem (z.B. eine Sende-Antenne und einen Sender) und ein HF-Empfangssystem (z.B. eine Empfangs-Antenne und einen Empfänger) und bevorzugt zusätzlich ein Gradientensystem (insbesondere ein unabgeschirmtes an einem sekundären Messplatz) und/oder ein Shimspulensystem. Das HF-Sendesystem und das HF-Empfangssystem können teilweise auch gemeinsame Komponenten aufweisen, beispielsweise eine gemeinsame HF-Sende-/Empfangsspule nutzen. Je nach Aufbau können das HF-Sende- und/oder HF-Empfangssystem einer Messeinrichtung eine Ganzkörperspule oder Lokalspule aufweisen. Bevorzugt kann die Messeinrichtung, je nach Art des Messplatzes, auch als mobile Messeinrichtung ausgebildet sein, um wie erwähnt einen hierfür vorgesehenen Messplatz zumindest temporär, während der Durchführung einer Magnetresonanztomographieaufnahme mit einer Messeinrichtung auszustatten. Ein Shimspulensystem oder ein Gradientensystem, welches auch als Shimspulensystem eingesetzt werden kann, ist besonders bei einem noch später genauer erläuterten "sekundären" Messplatz von Vorteil, da es dort ungewollte Inhomogenitäten im Streumagnetfeld kompensieren kann.

Der mobile Aspekt der Messeinrichtung kann so weit gehen, dass die Messeinrichtung so gestaltet ist, dass sie in einer Hand gehalten bzw. getragen werden kann. Bevorzugt sind ihre Abmessungen kleiner als 20 cm x 20 cm x 20 cm und ihr Gewicht liegt bevorzugt unter 5 kg. Dies ist durchaus realisierbar, da wie oben bereits gesagt worden ist, auf Abschirmungen zum größten Teil verzichtet werden kann. Alleine eine HF-Abschirmung ist für die mobile Messeinrichtung an fast allen Seiten vorteilhaft. Ein Bereich sollte dabei nicht mit dieser HF-Abschirmung abgeschirmt sein, damit dieser als Messbereich dienen kann. Trotz der Abschirmung der Messeinrichtung selber ist es von Vorteil, den Patienten mit der Messeinrichtung in einem RF-abgeschirmten Raum oder einer entsprechend abgeschirmten Kabine zu untersuchen.

Diese Messeinrichtung kann von einer (ggf. übergeordneten) Steuereinrichtung bezüglich des Ablaufs bzw. der Zeitpunkte von Messschritten gesteuert werden, wobei eine solche Steuereinrichtung bevorzugt so gestaltet ist, dass eine Koordinierung mehrerer Messeinrichtungen realisiert werden kann. Weiter unten wird eine solche bevorzugte Steuereinrichtung genauer beschrieben. In diesem Rahmen ist eine zentral koordinierte Steuerung sehr von Vorteil. Es ist insbesondere in dieser Hinsicht besonders bevorzugt, einen Abgleich von Gradienten der einzelnen Messungen durchzuführen, so dass diese sich z.B. nicht gegenseitig beeinflussen.

Ein erfindungsgemäßes Verfahren zur Messung von Rohdaten für eine Magnetresonanztomographieaufnahme, insbesondere für die Magnetresonanztomographiebildgebung, umfasst die folgenden Schritte:
- Positionieren zumindest eines Objekts in einem Messplatz eines erfindungsgemäßen Magnetresonanztomographie-Systems. Dieses Objekt kann insbesondere ein Patient bzw. ein Proband aber auch ein unbelebter Körper sein, z.B. ein Phantom.
- Erzeugen eines Grundmagnetfelds mit der Grundfeldmagnetanordnung des Magnetresonanztomographie-Systems.
- Messung der Rohdaten. Damit ist eine übliche MR-Anregung zumindest eines interessierende Bereichs (RoI) des Objekts mit geeigneten HF-Signalen gemeint und eine Erfassung (Akquisition) der dadurch induzierten MR-Signale aus dem RoI. Eine Ortskodierung kann mit Inhomogenitäten des Grundmagnetfeldes und/oder durch zusätzliche Gradientenfelder erreicht werden. Diese Rohdaten dienen nach entsprechender Verarbeitung bevorzugt zur Bildgebung, d.h. zur Erzeugung von Magnetresonanzbilddaten, können aber durchaus auch anderen Ergebnissen dienen.

Die Erfindung betrifft, wie dies später noch an verschiedenen Ausführungsvarianten näher erläutert wird, die Verwendung des Streumagnetfeldes eines Grundmagnetfeldes (welches an sich für einen "primären" Messplatz erzeugt wird und hier seinen "Hauptfeldbereich" aufweist) zur Durchführung einer Messung von Rohdaten an einem "sekundären" Messplatz, welcher im Streufeld bzw. Streufeldbereich diese Grundmagnetfelds angeordnet wird. Als Hauptfeldbereich wird hier das Grundmagnetfeld im bestimmungsgemäßen Messbereich oder "Sichtfeld" (engl: Field of View; FoV) angesehen. Dieser Messbereich kann auch als "Bildgebungsvolumen" bezeichnet werden. Als Hauptfeldbereich wird bevorzugt das Grundmagnetfeld in einer Kugel mit einem Durchmesser von ca. 50 cm im Zentrum des Messbereichs angesehen und der übrige Bereich des Magnetfelds als Streufeldbereich.

Zur Ansteuerungen aller Komponenten der Grundfeldmagnetanordnung und der Messeinrichtung(en) des Magnetresonanztomographie-Systems, kann dieses eine Steuereinrichtung aufweisen, wobei wie erwähnt diese vorzugsweise auch so ausgebildet sein kann, dass die Messungen an verschiedenen Messplätzen koordiniert ablaufen.

Gemäß einer bevorzugten Ausführungsform ist die Steuereinrichtung eines MR-Systems zur Koordinierung der Messeinrichtungen so ausgestaltet, dass sie das Timing von Messungen an unterschiedlichen Messplätzen so koordiniert, dass keine Interferenzen zwischen diesen Messungen auftreten. Insbesondere lässt sich dadurch der Einfluss von Feldfluktuationen (z.B. durch Gradienten) auf Messungen minimieren.

Ein Teil der Komponenten der Steuereinrichtung, insbesondere Komponenten die für eine Koordinierung der Messungen sorgen, können ganz oder teilweise in Form von Softwaremodulen in einem Prozessor einer entsprechenden Steuereinrichtung eines MR-Systems realisiert werden. Eine weitgehend softwaremäßige Realisierung solcher Komponenten hat den Vorteil, dass auch schon bisher verwendete Steuereinrichtungen auf einfache Weise durch ein Software-Update nachgerüstet werden können, um auf die erfindungsgemäße Weise zu arbeiten.

Die Steuereinrichtung kann auch mehrere Teil-Steuereinrichtungen umfassen, die zum Beispiel verschiedenen Messplätzen und/oder Messeinrichtungen zugeordnet sind und sich vorzugsweise zur Koordinierung untereinander verständigen können und/oder durch einen übergeordnete Master-Steuereinheit koordiniert werden. Insbesondere kann also eine Messeinrichtung jeweils auch eine eigene Steuereinheit aufweisen, insbesondere auch mit einer Sequenzsteuereinheit und ggf. sogar einer eigenen Rekonstruktionseinheit.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Ein bevorzugtes Magnetresonanztomographie-System ist wie erwähnt dazu ausgelegt, dass an zumindest zwei der Messplätze in dem gemeinsamen Grundmagnetfeld gleichzeitig Messungen für Magnetresonanztomographieaufnahmen durchgeführt werden können. Dies bedeutet, dass gleichzeitig zwei Objekte an diesen Messplätzen untersucht werden oder ggf. andere Messungen durchgeführt werden können. Dafür ist jedoch nicht zwingend erforderlich, dass gleichzeitig eine Rohdatenmessung, also HF-Anregung und/oder Rohdatenakquise erfolgen muss, diese kann auch in geeigneter Weise koordiniert sein, z.B. verschachtelt sein.

Insofern ist also eine "gleichzeitige Durchführung von Magnetresonanztomographieaufnahmen" so zu verstehen, dass zu einer "Magnetresonanztomographieaufnahme" (manchmal auch einfach "Bildgebung" genannt) der Zeitraum gehört, in den alle Maßnahmen fallen, die zu einer typischen MR-Messung gehören: Positionierung des Patienten, ggf. Applikation eines Kontrastmittels, Applikation einer Pulssequenz, Messung der MR-Signale, und ggf. Rekonstruktion eines Bildes, entlassen des Patienten aus dem Untersuchungsraum etc., also alle Vorgänge, die das MR-System belegen. Die Rohdatenmessung umfasst hierbei - als ein Teil der Magnetresonanztomographieaufnahmen - nur die Applikation einer Pulssequenz bzw. eine HF-Anregung und eine Messung der dadurch induzierten MR-Signale. Die Erfassung der MR-Signale selbst wird auch als "Rohdatenakquise" bezeichnet.

Eine gleichzeitige Durchführung von Magnetresonanztomographieaufnahmen bedeutet also, dass sich die Zeiträume von Bildgebungen an verschiedenen Messplätzen zeitlich überdecken bzw. überlappen können. Dabei kann es von Vorteil sein, wenn innerhalb dieses gemeinsam beanspruchten Zeitraums aktive Prozesse, die das Magnetfeld oder eine andere Messung beeinflussen können, getrennt voneinander durchgeführt werden. Passive Prozesse, also Prozesse, die keinen Einfluss auf eine andere Messung haben, wie z.B. ein Vorschub eines Patiententisches oder die Gabe eines Kontrastmittels, können wie erwähnt in der Regel durchaus gleichzeitig erfolgen. Das schließt aber nicht aus, dass je nach Aufbau und Anordnung der Messplätze zueinander auch aktive Prozesse oder die Messung von Rohdaten an verschiedenen Messplätzen gleichzeitig erfolgen kann.

Beispielsweise könnte wechselweise gemessen werden, um eine wechselseitige Störung zu vermeiden, d.h. das immer an einem der Messplätze eine Messung durchgeführt wird (also HF-Pulse ausgesendet und Rohdaten akquiriert werden) während an einem anderen der Messplätze gerade ein Patient positioniert wird, eine Verschiebung der Position des Patienten erfolgt oder ein Kontrastmittel verabreicht wird. Bekanntermaßen blockiert ja auch die Vorbereitungs- und Nachbereitungszeit des Untersuchungsablaufs das Magnetresonanztomographie-System über einen nicht unerheblichen Zeitraum, der so effektiver genutzt werden könnte.

Ein bevorzugtes Magnetresonanztomographie-System gemäß einer ersten bevorzugten Hauptvariante, die nicht Teil der vorliegenden Erfindung ist, umfasst eine besondere Grundmagnetfeldanordnung und mehrere Messplätze innerhalb des "Hauptfeldbereich" des Grundmagnetfelds dieser Grundfeldmagnetanordnung bzw. sogar innerhalb der Grundfeldmagnetanordnung selber.

Diese Grundfeldmagnetanordnung weist mehrere räumlich voneinander getrennte (aktive) Grundfeldmagnet-Segmente auf, um (im Betrieb) jeweils ein bestimmungsgemäßes Magnetfeld mit einer definierten Segment-Hauptfeldrichtung zu erzeugen.

Die Grundfeldmagnet-Segmente stellen dabei Teile der Grundfeldmagnetanordnung dar und umfassen mindestens einen Grundfeldmagneten, der durch zumindest eine Magnetspule definiert ist. Es können aber auch mehrere einzelne Grundfeldmagneten bzw. Magnetspulen zu einem Grundfeldmagnet-Segment zusammengeschlossen sein. Auch wenn ein Joch in einem Grundfeldmagneten nicht ausgeschlossen ist, ist es doch zumindest für einige Anwendungen von Vorteil, die Grundfeldmagneten so leicht wie möglich zu gestalten, also auf ein Joch zu verzichten.

D.h. die Magnetspulen der Grundfeldmagnet-Segmente sind bevorzugt eisenkernfrei, gegebenenfalls mit einem Freiraum im Kernbereich ausgebildet, bzw. die Grundfeldmagnet-Segmente sind bevorzugt eisenjochfrei. Die Grundfeldmagneten können als herkömmliche Elektromagneten oder als supraleitende Elektromagneten ausgestaltet sein. Da die Magnetspulen der Grundfeldmagneten hier im Vordergrund stehen, können die Grundfeldmagnet-Segmente auch insbesondere als "Grundfeldmagnet-Spulensegmente" bezeichnet werden.

Ein "bestimmungsgemäßes" Magnetfeld (sowie ein bestimmungsgemäßes Grundmagnetfeld) ist dasjenige Magnetfeld, dass sich bei einem bestimmungsgemäßen Betrieb eines Magneten, also wenn ein Strom durch den Magneten fließt, ausbildet. Die Form ist durch das Design des Magneten fest vorgegeben, wobei die Stärke des bestimmungsgemäßen Magnetfelds bei unterschiedlichen Stromstärken in der Stärke skaliert. Da im technischen Bereich der MR-Systeme in der Regel Helmholtz-Spulen verwendet werden, ist das bestimmungsgemäße Magnetfeld eines Grundfeldmagneten meist das eines Solenoiden. Das bestimmungsgemä-ße Magnetfeld eines Grundfeldmagnet-Segments bzw. der Grundfeldmagnetanordnung kann komplexer sein, zumindest wenn das Grundfeldmagnet-Segment eine andere Form als die einer Helmholtz-Spule hat.

Die "Hauptfeldrichtung" eines Magnetfelds eines Magneten wird durch denjenigen Vektor angezeigt, der den Magnetfeldverlauf innerhalb des Magneten charakterisiert. Es wird also nicht das Streufeld betrachtet, sondern das Feld, welches im Rahmen der bekannten MR-Systeme primär relevant ist. Bei einem solenoiden Magneten würde die Hauptfeldrichtung senkrecht zu der Stirnfläche des Magneten (Solenoiden) stehen, bei einem toroiden Magneten würde die Hauptfeldrichtung des Magnetfeldes im Inneren des Magneten eine kreisförmige Bahn anzeigen. Bei einem beliebig geformten Magneten gibt die Hauptfeldrichtung den Verlauf des resultierenden Magnetfeldvektors des stärksten Teils des Magnetfeldes (ohne das Streufeld) wieder. Die erwähnte Segment-Hauptfeldrichtung ist die Hauptfeld-richtung eines Magnetfelds eines Grundfeldmagnet-Segments.

In einer nicht zur Erfindung gehörenden Grundfeldmagnetanordnung sind zumindest zwei der Grundfeldmagnet-Segmente so zueinander angeordnet, dass die Segment-Hauptfeldrichtungen ihrer bestimmungsgemäßen Magnetfelder unter einem Ablenkwinkel zueinander verlaufen. Dieser Ablenkwinkel ist dabei selbstverständlich größer als 0°, da ja sonst keine Ablenkung erreicht werden würde. Die betreffenden Grundfeldmagnet-Segmente sind des Weiteren so zueinander angeordnet, dass die bestimmungsgemäßen Magnetfelder der Grundfeldmagnet-Segmente in einem bestimmungsgemäßen Grundmagnetfeld (der Grundfeldmagnetanordnung) resultieren. Die Grundfeldmagnet-Segmente bilden also im Betrieb gemeinsam das Grundmagnetfeld aus.

Die Grundfeldmagnet-Segmente sind dabei so angeordnet, dass das Grundmagnetfeld eine Grundmagnet-Hauptfeldrichtung mit einem ringförmigen Verlauf aufweist.

Dabei bezeichnet "ringförmig" einen geschlossenen Verlauf, vorzugsweise in einer einzigen Raumebene, bevorzugt in einer Kreisform oder zumindest einer Form mit abgerundeten Ecken. Ein solches ringförmiges Magnetfeld hat ein geringeres Streufeld als das Magnetfeld eines herkömmlichen Solenoiden. Das Streufeld wird kleiner, umso mehr Grundfeldmagnet-Segmente verwendet werden bzw. je ausgedehnter die Grundfeldmagnet-Segmente in Richtung des Magnetfeldverlaufs sind. Verglichen mit einem C-Magneten ist das Gesamtgewicht der bevorzugten Grundfeldmagnetanordnung um ein vielfaches kleiner, insbesondere da hier auf ein Eisenjoch verzichtet werden kann, dass in C-Magneten üblicherweise benötigt wird, um das Streufeld zu reduzieren.

Vorzugsweise kann also eine parallele Durchführung von Magnetresonanztomographieaufnahmen an mehr als einem Messplatz innerhalb des gemeinsamen ringförmigen Grundmagnetfelds (also in dessen Hauptfeldbereich) erfolgen.

Hierbei kann es sich um ein "torodiales" Magnetfeld handeln. Als ein "torodiales" Magnetfeld, also ein Magnetfeld, welches ähnlich zu dem Magnetfeld eines Toroiden ist, werden neben (im Wesentlichen kreisförmigen) toroidförmigen Magnetfeldern selbst insbesondere auch andere in sich geschlossene Magnetfelder angesehen, welche die Form einer Ellipse, eines Rechtecks mit abgerundeten Ecken oder eine Form aus Kreissegmenten und "eingefügten" geraden Passagen haben (insbesondere die Form einer einfachen Rennbahn mit 180° Kehren und zwei gegenläufigen geraden Strecken).

Eine solche Grundfeldmagnetanordnung kann mindestens drei Grundfeld-magnet-Segmente (bevorzugt mindestens vier, weiter bevorzugt mindestens sechs oder insbesondere bevorzugt mindestens acht) umfassen, welche so angeordnet sind, dass die Grundmagnet-Hauptfeldrichtung die Form eines ebenen Rings, bevorzugt im Wesentlichen eines Toroids oder eine toroid-ähnliche Form, insbesondere die Form eines Toroids mit eingefügten geraden Passagen, aufweist (siehe oben). Dies bedeutet, dass die Segment-Hauptfeldrichtungen der Magnetfelder der Grundfeldmagnet-Segmente alle in einer einzigen gemeinsamen Raumebene liegen.

Der Ablenkwinkel der Segment-Hauptfeldrichtungen beträgt zwischen zumindest zwei benachbarten Grundfeldmagnet-Segmenten mindestens 5°, bevorzugt mindestens 30°, besonders bevorzugt mindestens 45°. Dies heißt, dass die Grundfeldmagnet-Segmente (bezüglich ihrer Segment-Hauptfeldrichtung) entsprechend zueinander verkippt angeordnet sind. Diesbezüglich ist bevorzugt, dass Grundfeldmagnet-Segmente mit einer ihrer Seitenwände einander zugewandt sind und die Wickel- bzw. Spulenebenen benachbarter Grundfeldmagnet-Segmenten zueinander verkippt sind. Die Seitenwände sind hier die seitlichen Wände in denen Stromleiter der Magnetwicklungen verlaufen. Man könnte die Seitenwände auch als Kanten bezeichnen. Als Seitenwände sind also nicht die Stirnseiten eines Magneten zu verstehen (aus denen das Magnetfeld austritt und die z. B. im Wesentlichen parallel zu einer Wickelebene einer Magnetspule des Grundfeldmagnet-Segments liegen), sondern die Seiten in einer Richtung quer zur Segment-Hauptfeldrichtung des Grundfeldmagnet-Segments.

Die Grundfeldmagnetanordnung kann zumindest eine Gruppe von Grundfeld-magnet-Segmenten umfassen, die sternförmig um zumindest eine Raumachse herum angeordnet sind, wobei jeweils eine Seitenwand bzw. Kanten des jeweiligen Grundfeldmagnet-Segments zu dieser Mittelachse zeigt. Diese Anordnung ist bevorzugt drehsymmetrisch, wobei besonders bevorzugt bei N Grundfeldmagnet-Segmenten (in einer Gruppe) eine Drehsymmetrie von 360°/N vorliegt. Bei sechs Grundfeldmagnet-Segmenten sähe die Grundfeldmagnetanordnung z.B. aus wie ein sechszackiger Stern. Eine Sternform kann aber auch eine teilweise regelmäßige Anordnung von Grundfeldmagnet-Segmenten umfassen, z.B. dass die Grundfeldmagnet-Segmente alle regelmäßig innerhalb eines Halbkreises angeordnet sind. Bevorzugt ist auch eine Anordnung von mehreren dieser teilweise regelmäßigen Sternformen um mehrere Mittelachsen bzw. Raumachsen, z.B. zwei halbkreisförmige Anordnungen, die sich etwas beabstandet gegenüberliegen, um insgesamt z.B. das bereits erwähnte Grundmagnetfeld in der Form eines Toroids mit eingefügten geraden Passagen zu erzeugen.

Die Grundfeldmagnetanordnung kann ein Grundfeldmagnet-Segment oder eine Gruppe von Grundfeldmagnet-Segmenten, welche aufgebaut ist, um die Grundmagnet-Hauptfeldrichtung des bestimmungsgemäßen Grundmagnetfelds um einen Gesamt-Ablenkwinkel von mindestens 60°, bevorzugt von mindestens 90°, weiter bevorzugt von mindestens 180° abzulenken. Bevorzugt verläuft dabei die Segment-Hauptfeldrichtung des Magnetfeldes dieses Grundfeldmagnet-Segments bzw. die resultierende Segment-Hauptfeldrichtung des resultierenden Magnetfeldes dieser Gruppe (also die Segmentgruppen-Hauptfeldrichtung) in einem Bogen, der eine Ablenkung um besagte Winkel darstellt. Dieses Grundfeldmagnet-Segment bzw. diese eine Gruppe von Grundfeldmagnet-Segmenten kann dazu verwendet werden, das Grundmagnetfeld gezielt zu führen. Insbesondere wenn wie oben beschrieben Grundfeldmagnet-Segmente sternförmig oder in ähnlicher Weise um eine Mittelachse angeordnet werden, um einen ringförmig geschlossenen Verlauf der Grundmagnet-Hauptfeldrichtung des Grundmagnetfelds zu erreichen, und die einzelnen Grundfeldmagnet-Segmente so aufgebaut sind, dass sie ein Magnetfeld erzeugen, welches in einer Raumrichtung senkrecht zu ihrer Segment-Hauptfeldrichtung im Wesentlichen homogen sind (also wenn die Grundfeldmagnet-Segmente beispielsweise in Form von Helmholtz-Spulen aufgebaut sind), kann dies zu einem Grundmagnetfeld führen, dessen Feldstärke in radialer Richtung (ausgehend von der Mittelachse) inhomogen ist, insofern dass es (stetig) abnimmt. Dies liegt daran, dass der Abstand zwischen den einzelnen Grundfeldmagnet-Segmenten nah an der Raumachse enger ist als in einem radial größeren Abstand von der Mittelachse. D.h. die Dichte der Feldlinien des resultierenden Grundmagnetfelds nimmt radial nach außen hin ab.

Insbesondere um dies auszugleichen und ein auch in radialer Richtung möglichst homogenes Grundmagnetfeld zu erreichen, kann ein Grundfeldmagnet-Segment (vorzugsweise jedes der Grundfeldmagnet-Segmente) eine Spulenwicklung aufweisen, die ein bestimmungsgemäßes Magnetfeld erzeugt, das zu einer Seite des Grundfeldmagnet-Segments, d.h. in einer Richtung quer zur Segment-Hauptfeldrichtung des Grundfeldmagnet-Segments, hin stärker wird. Dazu ist die Spulenwicklung bevorzugt so gestaltet, dass der Durchmesser einer Wicklung in zumindest einer Raumrichtung, verglichen mit ihrer benachbarten Wicklung, abnimmt und ihr Mittelpunkt dichter an einer Seite des ringförmigen Grundmagnetfelds liegt. Das Magnetfeld dieses einzelnen Grundfeldmagnet-Segments wird also bevorzugt zum Außenrand der Grundfeldmagnetanordnung (z.B. zum Außenrand der Kreisform, insbesondere Toroidform) hin stärker, um die ansonsten auftretende radiale Inhomogenität des insgesamt resultierenden Grundmagnetfelds möglichst gut auszugleichen. Man kann bezüglich der Grundfeldmagnet-Segmente auch sagen, dass die Wicklungen nach und nach zur äußeren Seitenwand eines Grundfeldmagnet-Segments hin tendieren, also der Außenseite der Ringform des Grundmagnetfeldes. Die genaue Art bzw. Topographie der Wicklung wird hierbei bevorzugt entsprechend an die Anordnung der Grundfeldmagnet-Segmente zueinander angepasst, um einen geeigneten Ausgleich der Inhomogenität zu erreichen.

Wie erwähnt umfasst eine nicht zur Erfindung gehörende Variante des Magnetresonanztomographie-Systems eine Mehrzahl von Messplätzen (mindestens zwei) innerhalb einer (gemeinsamen) Grundfeldmagnetanordnung bzw. innerhalb eines Hauptfeldbereichs des gemeinsamen Grundmagnetfelds. Diese Messplätze sind dabei, insbesondere bei den zuvor genannten Grundfeldmagnetanordnungen, vorzugsweise zwischen jeweils zwei Grundfeldmagnet-Segmenten und/oder in einem Patiententunnel innerhalb eines Grundfeld-magnet-Segments angeordnet.

Ähnliche Magnetresonanztomographiesysteme, die mehrere Messplätze im Hauptmagnetfeld nutzen sind aus US5614880A, JPS61159950A, JPH10234704A, EP0311294A2 und DE19610266A1 bekannt.

Wie bereits oben erwähnt kann die Grundfeldmagnetanordnung eines Magnetresonanztomographie-Systems ein Grundfeldmagnet-Segment oder eine Gruppe von Grundfeld-magnet-Segmenten aufweisen, welche aufgebaut ist, um die Grundmagnet-Hauptfeldrichtung des bestimmungsgemäßen Grundmagnetfelds um einen Gesamt-Ablenkwinkel von mindestens 60°, bevorzugt von mindestens 90°, weiter bevorzugt von mindestens 180° abzulenken. Eine solche Grundfeldmagnetanordnung kann verwendet werden, um die Grundmagnet-Hauptfeldrichtung von einem Messplatz zu einem anderen Messplatz um 180° abzulenken, so dass die Grundmagnet-Hauptfeldrichtung an diesen Messplätzen gegenläufig ist.

Ein Grundfeldmagnet-Segment oder eine Gruppe von Grundfeldmagnet-Segmenten kann unter oder über zumindest einem Messplatz oder an zumindest einer Seite eines Messplatzes angeordnet sein, wobei vorzugsweise dieses Grundfeldmagnet-Segment oder die Gruppe von Grundfeld-magnet-Segmenten an zwei unterschiedliche Messplätze angrenzt.

Ein Grundfeldmagnet-Segment (z.B. ein Grundfeldmagnet) kann auch bevorzugt als Wand ausgebildet und/oder in einer Wand zwischen zwei Messplätzen angeordnet sein.

Eine Grundfeldmagnetanordnung kann eine Gruppe von in einem Halbkreis regelmäßig angeordneten Grundfeldmagnet-Segmenten aufweisen, wobei diese Gruppe das Grundmagnetfeld um 180° ablenkt, und ein weiteres Grundfeldmagnet-Segment, welches mittig senkrecht zu diesem Halbkreis angeordnet ist (als quasi auf diesem steht) und sich zwischen den beiden Messplätzen befinden kann. Dies wird später noch an einem Beispiel genauer erläutert.

Diese erste Hauptvariante, die nicht zur Erfindung gehört, lässt im Vergleich mit der traditionellen Magnetresonanztomographie, die auf homogene Grundmagnetfelder mit rechtwinkligen und parallelen Feldlinien basieren, Variationen in der Technik der Bildaufnahme, insbesondere der Signalcodierung, und der Bildrekonstruktion zu. Hier folgen die Signalcodierung und die Bildrekonstruktion den "Isofrequenzflächen", also Flächen mit gleicher Frequenz der aufgenommenen Bereiche. Diese Isofrequenzflächen sind in einem toroidalen Magnetfeld gekrümmt und folgen Flächen mit jeweils gleicher Magnetfeldstärke.

Im Folgenden wird eine zweite Hauptvariante, die die Erfindung darstellt und in Anspruch 1 definiert ist, genauer beschrieben, die das Streufeld eines MR-Systems zur Messung nutzt.

Ein bevorzugtes Magnetresonanztomographie-System umfasst hierzu die folgenden Komponenten:
- Zumindest einen primären Messplatz (mit einer Infrastruktur für MR-Messungen, zumindest Messspulen) der innerhalb der Grundfeldmagnetanordnung liegt, insbesondere innerhalb eines Grundfeldmagneten oder zwischen zwei Grundfeldmagneten, z.B. den Wicklungen eines C-Magneten. Bei der Grundfeldmagnetanordnung handelt es sich in der Regel um einen klassischen Aufbau wie ein Solenoid oder C-Magnet, wie er in herkömmlichen MR-Systemen eingesetzt wird, wobei auf die Abschirmung des Grundmagnetfeld (zumindest teilweise) verzichtet wird. In diesem Fall gibt es üblicherweise nur einen primären Messplatz. Dies schließt aber nicht aus, dass auch eine Grundfeldmagnetanordnung gemäß einer Ausführungsform der zuvor erläuterten ersten Hauptvariante mit mehreren Messplätzen handeln kann, sofern deren Streumagnetfeld ausreicht.
- Zumindest einen sekundären Messplatz (mit einer - wie oben erwähnt - zumindest temporären, bei anderen bevorzugten Ausführungsbeispielen aber auch permanenten, eigenen Infrastruktur für MR-Messungen, zumindest mit Messspulen) der außerhalb der Grundfeldmagnetanordnung liegt. Bevorzugt umfasst das MR-System dabei mindestens zwei sekundäre Messplätze.

Gemäß der Erfindung befindet sich also der sekundäre Messplatz in einem Streufeld-Bereich der Grundfeldmagnetanordnung und nutzt somit das Streufeld des Grundmagnetfelds. Beispielsweise kann dies bei einem Solenoiden als Grundfeldmagneten dadurch erreicht werden, dass der Grundfeldmagnet wie erwähnt keine oder nur eine eingeschränkten Abschirmung aufweist und sich die sekundären Messplätze neben dem Grundfeldmagneten, in dessen Streufeld befinden. Zumindest für einige MR-Messungen reicht dieses Streufeld aus, so dass parallel zu einer "Hauptmessung" an dem primären Messplatz weitere Messungen an den sekundären Messplätzen erfolgen können. Ein nicht abgeschirmter Grundfeldmagnet ist billiger und kleiner als ein abgeschirmter, so dass mit einem Verzicht auf eine (starke) Abschirmung ein finanzieller Vorteil hinzukommt.

Für die sekundären Messplätze gilt ähnliches wie oben für das toroidale Magnetfeld gesagt wurde, mit dem Unterschied, dass keine zusätzlichen Grundfeldmagnete bzw. Grundfeldmagnet-Segmente verwendet werden müssen.

Die US7355499B2 zeigt ein Magnetresonanzsystem mit zwei Messplätzen, wobei einer der Messplätze außerhalb der Bohrung, also im Streufeld liegt. Ein Hilfsmagnet wird außen an die Bohrung angesetzt um die Homogenität des B0 Feldes auch noch außerhalb der Bohrung zu gewährleisten.

Erfindungsgemäß sind die resultierenden Feldvektoren der bestimmungsgemäßen Magnetfeldverläufe (was weiter oben einer Segment-Hauptfeldrichtung entspricht, nur dass hier nun das Streufeld des Grundmagnetfeldes gemeint ist) an mindestens einem sekundären Messplatz um mindestens 30° zum bestimmungsgemäßen Grundmagnet-Hauptfeldverlauf (z.B. das Hauptfeld eines Solenoiden) an der Position des primären Messplatzes geneigt, bevorzugt entgegengesetzt.

Ein Vorteil dieser Ausführungsform bzw. zweiten Hauptvariante ist, dass einfach das Streumagnetfeld für zusätzliche Untersuchungen verwendet werden kann, welches sich bei einer primären Untersuchung sowieso um den Scanner herum ausbildet. Im Übrigen können zusätzlich die Kosten für eine Abschirmung des Grundfeldmagneten gespart werden, da ja gerade das Streumagnetfeld genutzt wird. Es kann möglicherweise eine "Lenkung" des Grundmagnetfeldes mittels weiterer Magneten (wie die Grundfeldmagnet-Segmente) vorgenommen werden, wie weiter oben beschrieben wurde.

Ein bevorzugtes Magnetresonanztomographie-System umfasst mindestens zwei an verschiedenen Seiten des primären Messplatzes angeordnete sekundäre Messplätze, welche bevorzugt in einer gemeinsamen Ebene mit dem primären Messplatz liegen. Alternativ oder zusätzlich umfasst ein bevorzugtes Magnetresonanztomographie-System mehrere sternförmig um den primären Messplatz herum angeordnete sekundäre Messplätze. Diese sekundären Messplätze liegen bevorzugt dort, wo der Verlauf des Streufeldes umgekehrt parallel zu einer Grundmagnet-Hauptfeldrichtung des bestimmungsgemäßen Grundmagnetfelds am primären Messplatz verläuft.

Bei einem bevorzugten Magnetresonanztomographie-System weist zumindest ein Messplatz, bevorzugt ein sekundärer Messplatz, eine Höhenverstellung auf, mittels derer die Höhe des gesamten Messplatzes und/oder des zu untersuchenden Objekts verstellt werden kann.

Bei einem bevorzugten Magnetresonanztomographie-System ist ein sekundärer Messplatz in einem anderen Raum (Untersuchungsraum) angeordnet als der primäre Messplatz und/oder der sekundäre Messplatz ist durch eine Wandung vom primären Messplatz getrennt. Eine Wandung kann dabei eine (Seiten-)Wand, eine Decke oder einen Boden sein. Es ist dabei bevorzugt, dass die Wandung zwischen dem primären Messplatz und dem sekundären Messplatz paramagnetisch ist und/oder die Wandungen um einen Messplatz einen Faradayschen Käfig bilden. Alternativ oder ergänzend stellt die Wandung eine akustische Trennung dar. Die Wandungen können auch als vorteilhafte Ausführungsform positionierbare ferromagnetische Elemente aufweisen, die für einen passiven Shim-Effekt entsprechend angeordnet werden können. Die Wandung ist bevorzugt undurchsichtig, um auch eine optische Abschirmung zu bieten.

Besonders bevorzugt ist eine mobile bzw. modulare Ausführungsform, bei der der primäre Messplatz und/oder der sekundäre Messplatz in unterschiedlichen Container-Modulen angeordnet sein können, wobei die Container nebeneinander bzw. aufeinander gestellt werden können. Die Wandungen der Container sind dabei vorzugsweise aus Aluminium oder Kunststoff gestaltet. Sie können aber auch die vorangehend beschriebenen positionierbaren ferromagnetischen Elemente aufweisen. Auch ein in die Wandungen ein- oder aufgebrachtes Kupfernetz ist als Faradayscher Käfig bevorzugt.

Bevorzugt ist bei einer Variante eines erfindungsgemäßen Magnetresonanztomographie-Systems eine Grundmagnet-Hauptfeld-richtung des Grundmagnetfelds im Bereich des primären Messplatzes senkrecht zu einer Bodenfläche ausgerichtet. Ein Grundfeldmagnet mit Patiententunnel würde also mit einer Stirnfläche auf dem Boden stehen und ein Patient würde stehend und nicht liegend untersucht. Bevorzugt befindet sich eine Anzahl sekundärer Messplätze im Bereich um den Grundfeldmagneten herum, wobei insbesondere auch dort Patienten stehend untersucht werden können. Eine solche Anordnung wäre auch im Boden versenkbar, was eine gute Abschirmung des Gesamtsystems nach außen mit sich führen würde.

Bei einem solchen Magnetresonanztomographie-System ist insbesondere wie erwähnt eine Höhenverstellung an den Messplätzen sinnvoll, um das zu untersuchenden Objekt im Messplatz zu positionieren.

Bevorzugt ist die Position eines sekundären Messplatzes so gewählt bzw. die oben beschriebene Grundfeldmagnetanordnung für einen Messplatz so ausgestaltet, dass an einem Messplatz das Magnetfeld (damit ist hier das Grundmagnetfeld und insbesondere auch dessen Streufeld gemeint) möglichst homogen ist. Um die Homogenität noch weiter zu verbessern, kann - insbesondere auch an einem sekundären Messplatz - ein Shimspulen-System eingesetzt werden. Hierzu kann auch auf Vorgehensweisen bzw. Prinzipien aus dem Stand der Technik an herkömmlichen Magnetresonanztomographie-Systemen zurückgegriffen werden, die gegebenenfalls in geeigneter Weise angepasst werden. Mit einem Gradientensystem, welches auch an jedem Messplatz vorhanden sein bzw. zu jeder Messeinrichtung gehören kann, können dann die gewünschten Gradientenfelder für die Messung angelegt werden.

Bei anderen bevorzugten Ausführungsformen kann aber auch eine vorliegende bekannte bzw. klar definierte Inhomogenität des Grundmagnetfeldes gezielt genutzt werden, z.B. zur Ortscodierung der Messdaten bzw. Rohdaten.

Hierzu kann insbesondere bei der oben genannten Grundfeldmagnetanordung mit dem ringförmig verlaufenden Grundmagnetfeldes ausgenutzt werden, dass bei einem ringförmigen Verlauf eines Magnetfeldes, wie oben erwähnt, ohne geeignete Gegenmaßnahmen in der Regel zum Mittelpunkt des Rings hin ein stärkeres Magnetfeld herrscht als am Rand. In der Regel ist die Abnahme der Feldstärke antiproportional zum Abstand vom Mittelpunkt des Rings. Da zur Ortskodierung im Rahmen von MR-Messungen in der Regel eine Inhomogenität des Magnetfeldes genutzt wird, welches aber bisher üblicherweise durch das Gradientenspulensystem eingestellt wird, kann eine durch den Aufbau der Grundfeldmagnetanordnung auftretende Inhomogenität des Grundmagnetfelds vorteilhaft genutzt werden. Zumindest ist es nicht unbedingt erforderlich, in der Richtung der Inhomogenität ein Gradientenfeld zu applizieren, wie es bisher im Stand der Technik getan wird.

Es sei ergänzend erwähnt, dass auch Kombinationen der verschiedenen Varianten möglich sind, also dass beispielsweise nur in einigen Raumrichtungen die Inhomogenität des Grundmagnetfelds ausgenutzt wird und in einigen Raumrichtungen zusätzliche Gradientenfelder eingesetzt werden oder dass dies auch von Messeplatz zu Messeplatz unterschiedlich gehandhabt wird.

Bevorzugt wird an einem sekundären Messplatz keine Ganzkörperspule verwendet (also Ganzkörperspulen in Form von HF-Spulen, Magnetspulen oder Gradientenspulen), sondern nur lokale Empfangs- und Sendespulen. Dies hat den Vorteil, dass die Wand des Messplatzes sehr dünn gestaltet werden kann. Bei Verwendung von einem Gradientensystem wären z.B. nur noch die Gradientenspulen in der Wand anzuordnen. Es ist auch von Vorteil, die Gradientenspulen in der Untersuchungsliege anzuordnen, was als "lokale orthogonale planare Gradientenspulen" bekannt ist. In einer weiteren bevorzugten Ausgestaltung weist ein Messplatz kein Gradientensystem auf, sondern eine Matrix aus Sende-Empfangsspulen, die neben ihrer Messfunktion zur Ortscodierung verwendet werden.

Gemäß einem bevorzugten Verfahren wird also im Rahmen der vorliegenden Erfindung auch zumindest ein Objekt in einem sekundären Messplatz (im Folgenden auch als "Satellitenmessplatz" bezeichnet) positioniert und Rohdaten für Magnetresonanztomographieaufnahmen an diesem sekundären Messplatz gemessen. Dies geschieht bevorzugt mit einer, vorzugsweise mobilen, erfindungsgemäßen Messeinrichtung. Besonders vorteilhaft ist, wenn an zumindest zwei Messplätzen gleichzeitig Messungen für Magnetresonanztomographieaufnahmen durchgeführt werden. Dabei ist es, wie oben bereits angedeutet, nicht zwingend erforderlich, dass gleichzeitig eine Rohdatenmessung oder HF-Anregung erfolgt, dies könnte auch wechselweise koordiniert sein. Gemäß einem besonders bevorzugten Verfahren wird dabei eine Inhomogenität des Grundmagnetfelds, insbesondere des Streumagnetfelds an einem sekundären Messplatz, zur Ortskodierung der Rohdaten genutzt.

Mit der Erfindung lassen sich auch vorzugsweise die Satellitenmessplätze so gestalten, dass sie speziell für bestimmte Untersuchungen optimiert sind. Beispielsweise kann ein sekundärer Messplatz speziell für eine Brustuntersuchung oder eine Kopfuntersuchung als Stuhl mit speziellen Halterungen ausgeformt sein, die Messspulen und/oder Gradientenspulen umfassen. Auch ist es bevorzugt, gynäkologische Messplätze oder Messplätze zur Prostatauntersuchung mit invasiven HF-Sonden und einem speziell auf die Anatomie angepassten Gradientensystem bereitzustellen. Weitere Beispiele wären neurologische Untersuchungen, Herzuntersuchungen, Nierenuntersuchungen, abdominale Untersuchungen, Angiographie und muskuloskelettale Untersuchungen. Des Weiteren können interventionale Untersuchungen gut in einem sekundären Messplatz durchgeführt werden.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine schematische Darstellung eines Magnetresonanztomographie-Systems gemäß dem Stand der Technik,
Figur 2 eine vereinfachte Darstellung eines nicht zur Erfindung gehörenden Magnetresonanztomographie-Systems mit einer ersten Grundfeldmagnetanordnung,
Figur 3 eine schematische Darstellung einer Grundfeldmagnetanordnung, wie sie im Magnetresonanztomographie-System gemäß Figur 2 genutzt werden kann,
Figur 4 eine etwas detailliertere Darstellung einer Grundfeldmagnetanordnung, wie sie im Magnetresonanztomographie-System gemäß Figur 2 genutzt werden kann,
Figur 5 ein Magnetresonanztomographie-System mit einer bevorzugten, nicht zur Erfindung gehörenden Grundfeldmagnetanordnung,
Figur 6 ein Magnetresonanztomographie-System mit einer bevorzugten, nicht zur Erfindung gehörenden Grundfeldmagnetanordnung,
Figur 7 ein weiteres Magnetresonanztomographie-System mit einer bevorzugten, nicht zur Erfindung gehörenden Grundfeldmagnetanordnung,
Figur 8 eine schematische Darstellung einer bevorzugten Spulenwicklung, wie sie beispielsweise in den Grundfeldmagnetanordnungen nach den Figuren 1 bis 7 genutzt werden kann,
Figur 9 eine detaillierte Darstellung einer Grundfeldmagnetanordnung ähnlich der in Figur 2, jedoch nun mit einer Spulenwicklung wie in Figur 8,
Figur 10 ein Magnetresonanztomographie-System mit zwei sekundären Messplätzen gemäß einem Ausführungsbeispiel der Erfindung,
Figur 11 ein weiteres Ausführungsbeispiel für ein Magnetresonanztomographie-System mit insgesamt vier sekundären Messplätzen gemäß der Erfindung,
Figur 12 ein weiteres Ausführungsbeispiel für ein Magnetresonanztomographie-System ebenfalls mit insgesamt vier sekundären Messplätzen gemäß der Erfindung,
Figur 13 ein weiteres Ausführungsbeispiel für ein Magnetresonanztomographie-System mit einer sternförmigen Anordnung von insgesamt sechs sekundären Messplätzen in aufrechter Position gemäß der Erfindung,
Figur 14 eine schematische, sehr einfache Block-Darstellung einer im erfindungsgemäßen Magnetresonanztomographie-System verwendbaren Messeinrichtung,
Figur 15 eine mobile bzw. modulare Ausführungsform eines MR-Systems in einer Containeranordnung.

In den folgenden Figuren sind nur für die Erfindung wesentliche oder zu ihrem Verständnis hilfreiche Elemente eingezeichnet.

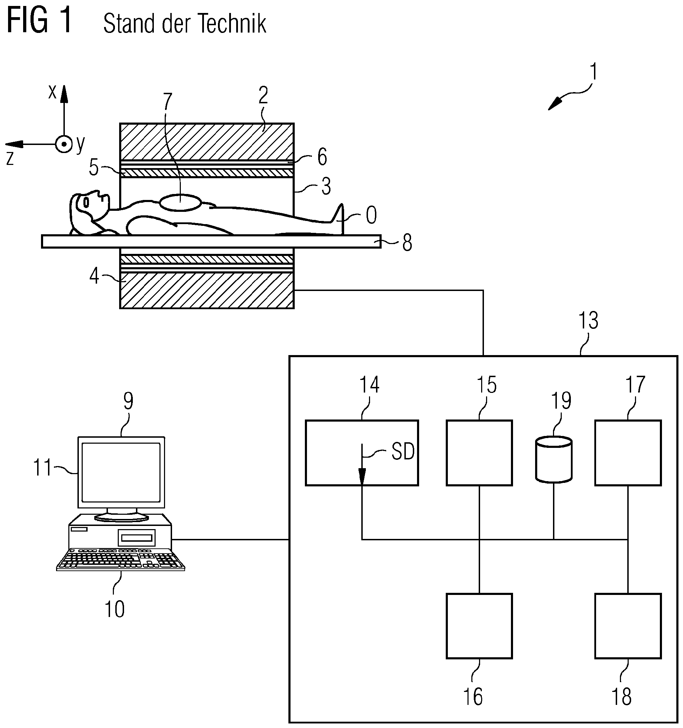

In Figur 1 ist grob schematisch ein Magnetresonanztomographie-System 1 dargestellt. Es umfasst zum einen den eigentlichen Magnetresonanzscanner 2 mit einem Messplatz 3 bzw. Untersuchungsraum 3, hier einem klassischen Patiententunnel 3, in dem auf einer Liege 8 ein Patient O oder Proband, d.h. das Untersuchungsobjekt O positioniert ist. In der Regel wird aber nicht der ganze Patient O gescannt, sondern nur eine interessierende Regionen innerhalb des Patienten O, d. h. nur aus diesem Bereich werden Rohdaten gemessen.

Der Magnetresonanzscanner 2 ist in üblicher Weise mit einem Grundfeldmagnetsystem 4, einem Gradientensystem 6 sowie einem HF-Sendeantennensystem 5 und einem HF-Empfangsantennen-system 7 ausgestattet. In dem dargestellten Ausführungsbeispiel handelt es sich bei dem HF-Sendeantennensystem 5 um eine im Magnetresonanzscanner 2 fest eingebaute Ganzkörperspule, wogegen das HF-Empfangsantennensystem 7 aus am Patienten bzw. Probanden anzuordnenden Lokalspulen besteht. Grundsätzlich können aber auch die Ganzkörperspule als HF-Empfangsantennensystem und die Lokalspulen als HF-Sendeantennensystem genutzt werden, sofern diese Spulen jeweils in unterschiedliche Betriebsweisen umschaltbar sind. Das Grundfeldmagnetsystem 4 ist hier in üblicher Weise so ausgebildet, dass es ein Grundmagnetfeld in Längsrichtung des Patienten erzeugt, d.h. entlang der in z-Richtung verlaufenden Längsachse des Magnetresonanzscanners 2. Das Gradientensystem 6 umfasst in üblicher Weise einzeln ansteuerbare Gradientenspulen, um unabhängig voneinander Gradienten in x-, y- oder z-Richtung schalten zu können. Zudem kann der Magnetresonanzscanner 2 (nicht dargestellte) Shimspulen aufweisen, die in üblicher Weise ausgebildet sein können.

Das Magnetresonanztomographie-System 1 weist weiterhin eine zentrale Steuereinrichtung 13 auf, die zur Steuerung des MR-Systems 1 verwendet wird. Diese zentrale Steuereinrichtung 13 umfasst eine Sequenzsteuereinheit 14. Mit dieser wird die Abfolge von Hochfrequenz-Pulsen (HF-Pulsen) und von Gradientenpulsen in Abhängigkeit von einer gewählten Pulssequenz oder einer Abfolge von mehreren Pulssequenzen zur Aufnahme mehrerer Schichten in einem interessierenden Volumenbereich des Untersuchungsobjekts innerhalb einer Messsitzung gesteuert. Eine solche Pulssequenz kann beispielsweise innerhalb eines Mess- oder Steuerprotokolls vorgegeben und parametrisiert sein. Üblicherweise sind verschiedene Steuerprotokolle für unterschiedliche Messungen bzw. Messsitzungen in einem Speicher 19 hinterlegt und können von einem Bediener ausgewählt (und bei Bedarf gegebenenfalls geändert) und dann zur Durchführung der Messung genutzt werden. Im vorliegenden Fall enthält die Steuereinrichtung 13 Pulssequenzen zur Messung der Rohdaten, also zur Anregung der MR-Signale für die Akquisition der Rohdaten.

Zur Ausgabe der einzelnen HF-Pulse einer Pulssequenz weist die zentrale Steuereinrichtung 13 eine Hochfrequenzsendeeinrichtung 15 auf, die die HF-Pulse erzeugt, verstärkt und über eine geeignete Schnittstelle (nicht im Detail dargestellt) in das HF-Sendeantennensystem 5 einspeist. Zur Steuerung der Gradientenspulen des Gradientensystems 6, um entsprechend der vorgegebenen Pulssequenz die Gradientenpulse passend zu schalten, weist die Steuereinrichtung 13 eine Gradientensystemschnittstelle 16 auf. Die Sequenzsteuereinheit 14 kommuniziert in geeigneter Weise, z. B. durch Aussendung von Sequenzsteuerdaten SD, mit der Hochfrequenzsendeeinrichtung 15 und der Gradientensystemschnittstelle 16 zur Ausführung der Pulssequenz.

Die Steuereinrichtung 13 weist außerdem eine (ebenfalls in geeigneter Weise mit der Sequenzsteuereinheit 14 kommunizierende) Hochfrequenzempfangseinrichtung 17 auf, um innerhalb der durch die Pulssequenz vorgegebenen Auslesefenster koordiniert mittels des HF-Empfangsantennensystems 7 Magnetresonanz-Signale zu empfangen und so die Rohdaten zu akquirieren.

Eine Rekonstruktionseinheit 18 übernimmt hier die akquirierten Rohdaten und rekonstruiert daraus Magnetresonanz-Bilddaten. Auch diese Rekonstruktion erfolgt in der Regel auf Basis von Parametern, die in dem jeweiligen Mess- oder Steuerprotokoll vorgegeben sein können. Diese Bilddaten können dann beispielsweise in einem Speicher 19 hinterlegt werden.

Wie im Detail durch ein Einstrahlen von HF-Pulsen und das Schalten von Gradientenpulsen geeignete Rohdaten akquiriert und daraus MR-Bilder oder Parameter-Karten rekonstruiert werden können, ist dem Fachmann grundsätzlich bekannt und wird daher hier nicht näher erläutert.

Eine Bedienung der zentralen Steuereinrichtung 13 kann über ein Terminal 11 mit einer Eingabeeinheit 10 und einer Anzeigeeinheit 9 erfolgen, über das somit auch das gesamte Magnetresonanztomographie-System 1 durch eine Bedienperson bedient werden kann. Auf der Anzeigeeinheit 9 können auch Magnetresonanztomographie-Bilder angezeigt werden, und mittels der Eingabeeinheit 10, gegebenenfalls in Kombination mit der Anzeigeeinheit 9, können Messungen geplant und gestartet und insbesondere Steuerprotokolle ausgewählt und gegebenenfalls modifiziert werden.

Solch ein Magnetresonanztomographie-System 1 und insbesondere die Steuereinrichtung 13 können darüber hinaus noch eine Vielzahl von weiteren, hier nicht im Einzelnen dargestellten, aber üblicherweise an derartigen Anlagen vorhandenen Komponenten aufweisen, wie beispielsweise eine Netzwerkschnittstelle, um das gesamte System mit einem Netzwerk zu verbinden und Rohdaten und/oder Bilddaten bzw. Parameterkarten, aber auch weitere Daten, wie beispielsweise patientenrelevante Daten oder Steuerprotokolle, austauschen zu können.

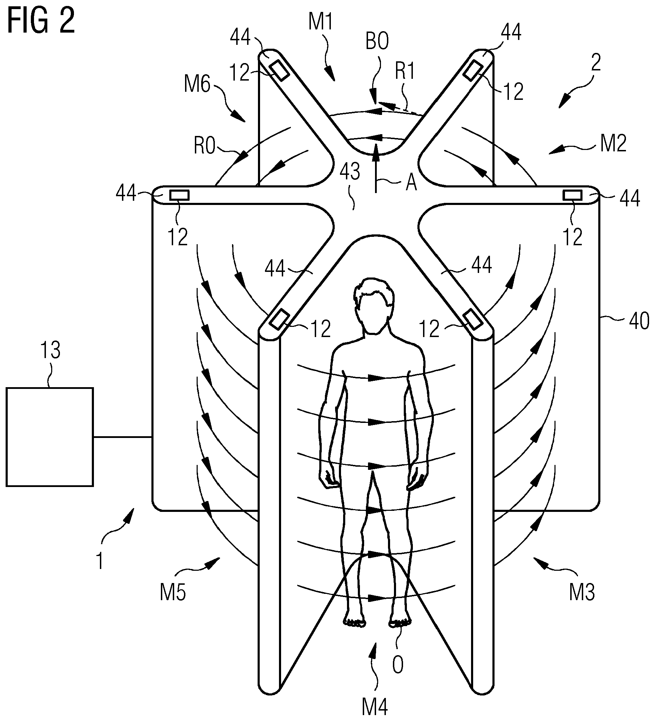

Figur 2 zeigt ein Ausführungsbeispiel für ein Magnetresonanztomographie-System 1 nach der ersten, nicht zur Erfindung gehörenden Hauptvariante mit einer bevorzugten Grundfeldmagnetanordnung 40, bei dem innerhalb eines Hauptfeldbereichs des Grundmagnetfelds dieser Grundfeldmagnetanordnung 40 mehrere Messplätze genutzt werden können.

Dargestellt ist hier ein Magnetresonanzscanner 2, dessen Funktion von einer Steuereinrichtung 13 gesteuert werden kann. Die Steuereinrichtung 13 kann dabei im Prinzip in ähnlicher Weise aufgebaut sein und die gleichen Komponenten aufweisen, wie die Steuereinrichtung 13 in einem herkömmlichen MR-System gemäß Figur 1. Ebenso kann diese auch ein geeignetes Terminal oder dergleichen aufweisen (was hier aber nicht dargestellt ist).

Die Grundfeldmagnetanordnung 40 des Magnetresonanzscanners 2 umfasst hier sechs (hier identische) Grundfeldmagnet-Segmente 44, die in dieser Ausführungsform sternförmig um eine Mittelachse A mit einer Drehsymmetrie von 60° angeordnet sind. Das durch Pfeile angedeutete Grundmagnetfeld B0 hat eine Grundfeld-Hauptrichtung R0, die in Form eines Kreises bzw. eines toroiden Magnetfeldes verläuft.

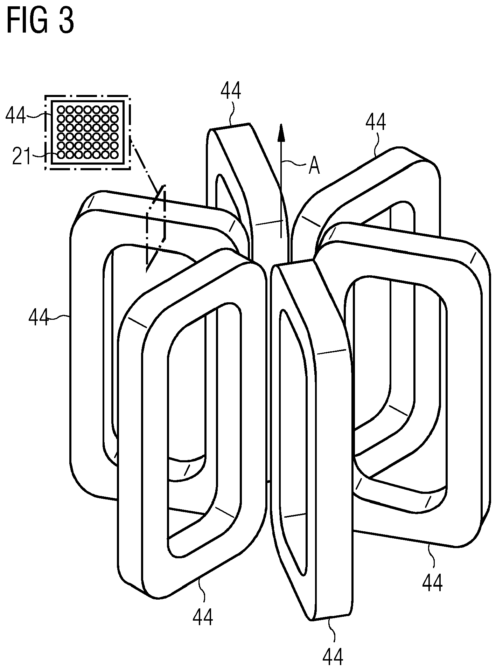

Figur 3 zeigt hierzu eine detaillierte schematische Darstellung der einzelnen Grundfeldmagnet-Segmente 44 der sternförmigen Grundfeldmagnetanordnung 40 in Figur 2.

Zu sehen sind hier sechs Helmholtz-Spulen als Grundfeldmagnet-Segmente 44 der Grundfeldmagnetanordnung 40. Sie sind mit einer Kante ihrer Spulenebene bzw. Wickelebene zur Mittelachse A hin ausgerichtet. Jedes einzelne Grundfeldmagnet-Segment 44 hat ein bestimmungsgemäßes Magnetfeld, welches dem eines (relativ kurzen) Solenoiden entspricht, d. h. die Segment-Hauptfeldrichtung R1 (in der Darstellung in Figur 2 nur bei einem der hinteren beiden Grundfeldmagnet-Segmente 44 gezeigt) des Magnetfelds, welches von einem einzelnen Grundfeldmagnet-Segment 44 erzeugt wird, würde jeweils senkrecht zur Stirnfläche des betreffenden Grundfeldmagnet-Segments 44 stehen und tangential zur Grundmagnet-Hauptfeldrichtung R0 verlaufen. Gemeinsam resultieren die einzelnen Magnetfelder der Grundfeldmagnet-Segmente 44 in dem in Figur 2 angezeigten Grundmagnetfeld B0 mit einer toroiden Grundmagnet-Hauptfeld-richtung R0, wobei die Segment-Hauptfeldrichtung R1 im Mittelpunkt der einzelnen Grundfeldmagnet-Segmente 44 jeweils "tangential" zur kreisförmigen Grundmagnet-Hauptfeldrichtung R0 steht. Das Grundmagnetfeld B0 nimmt in radialer Richtung nach außen hin ab, ist jedoch in der Höhe homogen.

Die Grundfeldmagnet-Segmente 44 der Grundfeldmagnetanordnung 40 sind so miteinander verschaltet, dass ein Gleichstrom von einem Grundfeldmagnet-Segment 44 in das nächste fließt, wobei die Stromrichtung durch die Magnetwicklungen stets dieselbe ist und sich durch den Stromfluss insgesamt das kreisförmiges Magnetfeld B0 ausbildet.

Ein signifikanter Vorteil einer solchen symmetrischen Anordnung ist die strukturelle Stabilität bei eingeschaltetem Grundmagnetfeld B0. Die magnetischen Kräfte zwischen den einzelnen Grundfeldmagnet-Segmenten 44 heben sich in Richtung der Grundmagnet-Hauptfeldrichtung R0 gegenseitig auf. Jedes Grundfeldmagnet-Segment 44 wird von seinen beiden Nachbarn mit jeweils der gleichen Kraft angezogen. Die resultierenden Kräfte wirken nach innen zur Säule 43 hin und können dort sehr gut durch entsprechende strukturelle Verstärkungen kompensiert werden.

In der linken oberen Ecke der Figur 3 ist vergrößert ein Schnitt durch ein Grundfeldmagnet-Segment 44 gezeigt. Zu sehen ist eine regelmäßige Anordnung von Stromleitern 21, die hier als Drähte eingezeichnet sind, jedoch durchaus einen komplexen Aufbau haben können, z.B. hohl sein können, um ein Kühlmittel hindurchzuleiten.

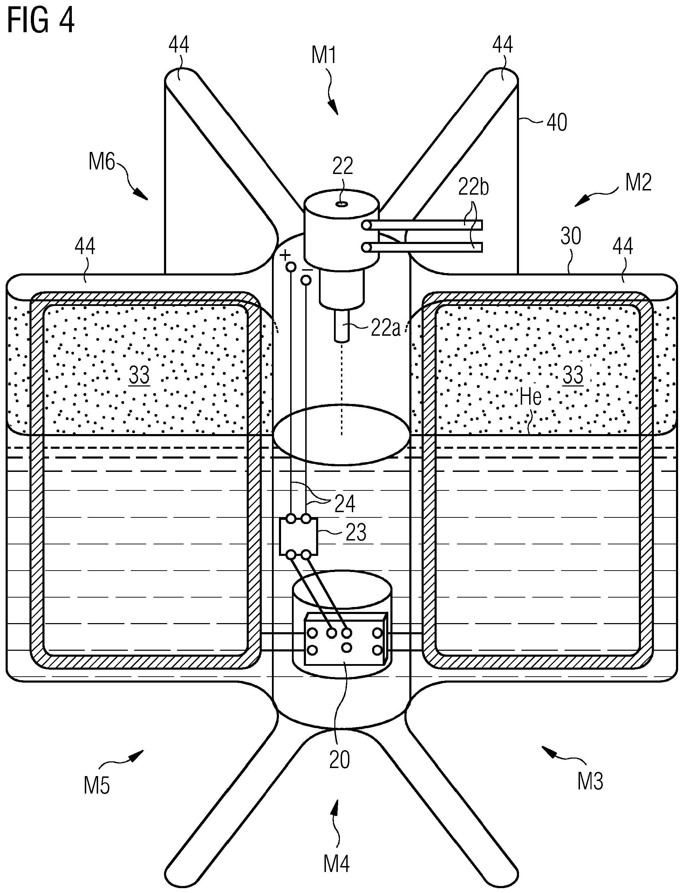

Ein solches Magnetresonanztomographie-System 1 mit einer Grundfeldmagnetanordnung 40 gemäß den Figuren 2 und 3 erlaubt Messungen an sechs verschiedenen Messplätzen M1, M2, M3, M4, M5, M6 (siehe Figur 2), wobei in dem dargestellten Beispiel gerade an Messplatz M4 eine Messung eines Objektes O stattfindet, wobei ein Patient hier aufrecht an senkrechten Wänden der Grundfeldmagnetanordnung 40 steht. Theoretisch könnten gleichzeitig Messungen an allen sechs Messplätzen M1, M2, M3, M4, M5, M6 stattfinden. Eine zentrale Säule 43 hält die Grundfeldmagnet-Segmente 44 an ihrem Platz und kann auch technische Komponenten umfassen, wie z.B. die elektrischen Verbindungen oder sogar die Stromversorgung (siehe z.B. Figur 4) .

An den Messplätzen M1, M2, M3, M4, M5, M6 können sich jeweils Messeinrichtungen 12 (nur jeweils symbolisch dargestellt) bzw. die hierzu am Messplatz M1, M2, M3, M4, M5, M6 jeweils erforderlichen Komponenten befinden, wie eine HF-Sendespule eines HF-Sendesystems, eine HF-Empfangsspule eines HF-Empfangssystem und/oder eine gemeinsame HF-Sende-/Empfangsspule. Ebenso können hierzu Gradienten- und/oder Shimspulen 6a gehören. Alle diese Komponenten können von der gemeinsamen Steuereinrichtung 13 koordiniert angesteuert werden.

Selbstverständlich kann ein Magnetresonanzscanner 2 auch mehr als sechs Messplätze M1, M2, M3, M4, M5, M6 aufweisen, seine Höhe kann geringer sein oder er ist zur Untersuchung kleiner Körperbereiche ausgelegt, z.B. für Kopfuntersuchungen oder Untersuchungen der Extremitäten, der weiblichen Brust, der Prostata, der Leber, Nieren oder von anderen Organen. Auch könnte die sternförmige Grundfeldmagnetanordnung 40 liegend positioniert sein.

Figur 4 zeigt ein Ausführungsbeispiel für ein Magnetresonanztomographie-System 1, mit einer supraleitenden Grundfeldmagnetanordnung 40. Dies könnte eine supraleitende Version der in Figur 2 gezeigten Ausführungsform sein. Zur besseren Darstellung des inneren Aufbaus sind die vorderen beiden Grundfeldmagnet-Segmente 44 nicht dargestellt. Zu sehen ist, dass die Grundfeldmagnetanordnung 40 mit Helium He gefüllt ist, welches teilweise flüssig und teilweise gasförmig ist. Das Referenzzeichen für das Helium He zeigt dabei auf den Flüssigkeitsspiegel. Die gesamte Grundmagnetfeldanordnung 40 ist von einer Gehäusewand 30 umgeben, die hier insbesondere eine thermische Isolierung aufweist, damit das Helium He im Gehäuseinnenraum 33 kalt und damit flüssig bleibt.

Solche eine Isolierung kann z.B. Vielschicht-Isolationsfolien oder thermisch leitfähige Abschirmungen gegen umgebende thermische Strahlung umfassen.

Oben in der Mitte der Grundfeldmagnetanordnung 40 ist eine Kühleinheit 22 angebracht, an deren Kühlfinger 22a ständig Helium He kondensiert und nach unten tropft. Durch eine Heliumleitung 22b kann der Heliuminhalt und der Druck in der Grundfeldmagnetanordnung 40 reguliert werden. Unten ist eine Magnetzuleitungsverteiler 20 (engl.: superconducting joints box) an einer Schalteinheit 23 zu sehen, die über Stromzuführungen 24 die Grundfeldmagnetanordnung 40 mit Strom versorgen kann. Die Schalteinheit 23 kann hier als persistenter Schalter verwendet werden, um einen stetig kreisenden Strom und damit ein permanentes Grundmagnetfeld B0 in der supraleitenden Grundfeldmagnetanordnung 40 zu erzeugen.

Es sind aber auch andere Kühlungsalternativen möglich, z.B. mittels Durchleitung von flüssigem Helium durch Hohlleiter der Magnete oder durch zusätzliche Kühlleitungen in gutem thermischen Kontakt mit den Magnetspulen. Es können in dem Aufbau der der Grundfeldmagnetanordnung 40 noch weitere Komponenten enthalten sein, die der Übersicht halber hier nicht eingezeichnet sind wie z.B. ein Quench-Detektor bzw. -Schutz, ein sogenannter "coil carrier" (Magnetformer) oder strukturelle Verstärkungen.

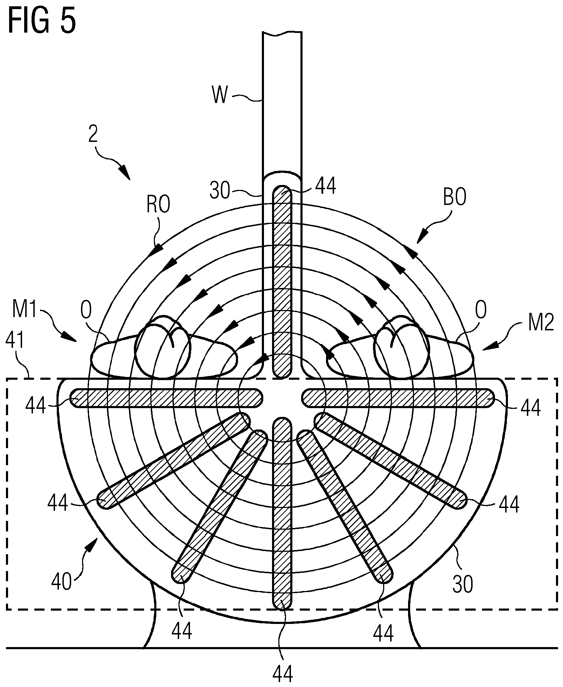

Figur 5 zeigt ein weiteres Ausführungsbeispiel für einen Magnetresonanzscanner 2 mit einer weiteren Grundfeldmagnetanordnung 40. Hier ist nur die untere Hälfte der Grundfeldmagnetanordnung 40 als eine Gruppe 41 von Grundfeldmagnet-Segmenten 44 sternförmig gestaltet und ein weiteres Grundfeldmagnet-Segment 44 ragt nach oben heraus und dient sowohl zur Führung des Grundmagnetfeldes B0 als auch als Teil einer Wandung W zwischen zwei Messplätzen M1, M2, auf denen sich hier zwei Patienten O befinden. In der Darstellung ist zu sehen, dass der untere Teil der Wandung W zwischen den beiden Patienten O durch die Gehäusewandung 30 des Magnetresonanzscanners 2 gebildet wird, in die das Grundfeldmagnet-Segment 44 zwischen den Messplätzen M1, M2 integriert ist. Die Wandung W kann nicht nur als ein Sichtschutz dienen, sondern auch als eine akustische Abschirmung bzw. eine HF-Abschirmung.

Das Grundmagnetfeld B0 dieses Magnetresonanzscanners 2 wird nach außen hin schwächer, was zur Ortscodierung genutzt werden kann, und ist in Längsrichtung (orthogonal zur Bildebene) homogen. Es ist im Grunde in den beiden Messplätzen M1, M2 von seiner Form her gleich, mit dem einzigen Unterschied, dass der Verlauf (in einer Richtung durch die Oberfläche, auf der der Patient O gelagert wird) umgekehrt ist. Auch hier können wie bei Figur 2 die Dimensionen des Magnetresonanzscanners 2 durchaus anders gewählt werden.

Die Grundmagnet-Hauptfeldrichtung R0 ist auch hier kreisförmig. Eine Besonderheit bei dieser Ausführungsform ist, dass die Patienten O nicht in einem engen Raum liegen, sondern frei zur Decke schauen können. Die durch die Krümmung in der Regel hervorgerufene Inhomogenität im Grundmagnetfeld B0 kann wie erwähnt zur Ortskodierung einer Raumrichtung verwendet werden, so dass zur Gesamtortskodierung lediglich Gradienten in die andere Raumrichtung appliziert werden müssen.

Diese Anordnung lässt wegen ihrer offenen Bauform und dem toroidalen Magnetfeld einen einfachen und wenig eingeschränkten Zugang zum Patienten zu. Durch den besonderen Aufbau werden Magnetkräfte wie bei der Figur 2 zum großen Teil kompensiert bzw. in Bereiche abgeleitet, die sich gut strukturell verstärken lassen.

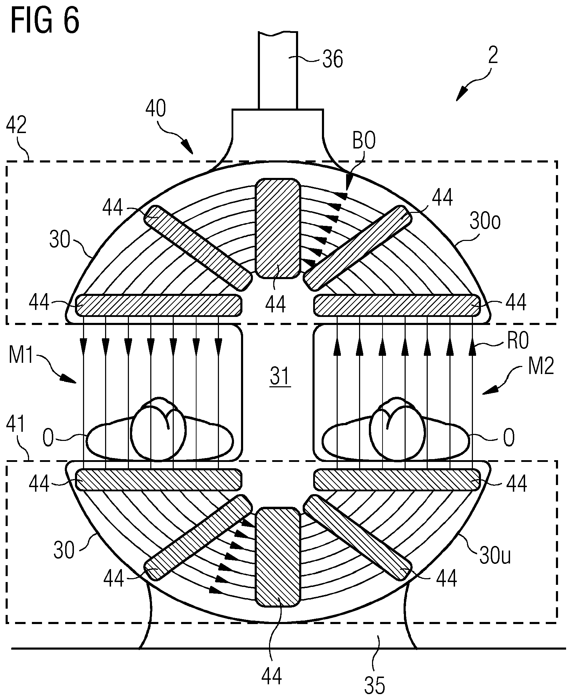

Figur 6 zeigt ein weiteres Ausführungsbeispiel für einen Magnetresonanzscanner 2 mit einer weiteren Grundfeldmagnetanordnung 40. Diese ist ähnlich zu der in Figur 5 aufgebaut, mit dem Unterschied, dass sich nun über und unter den beiden Messplätzen M1, M2 eine Gruppe 41, 42 von Grundfeldmagnet-Segmenten 44 befindet. Wie an dem eingezeichneten Verlauf der Magnetfeldlinien zu sehen ist, ist der Verlauf des bestimmungsgemäßen Grundmagnetfelds B0 hier sehr homogen im Bereich der Messplätze M1, M2.

Das Gehäuse 30 umfasst hier einen oberen und einen unteren halbzylinderförmigen Gehäuseabschnitt 30u, 30o, jeweils mit einem Querschnitt in der Form eines 180° Kreissegmentes, in denen jeweils eine Gruppe 41, 42 von Grundfeldmagnet-Segmenten 44 untergebracht ist. Die Gruppen 41, 42 von Grundfeld-magnet-Segmenten 44 werden mittels eines Mittelstegs bzw. Abgrenzungselements 31, welches Teil des Gehäuses 30 ist, voneinander beabstandet gehalten, wobei die Halbzylinder der Gehäuseabschnitte 30u, 30o jeweils mit ihrer flachen Seite aufeinander zuweisen, wodurch zwei Messeplätze M1, M2 zwischen den Gehäuseabschnitten 30u, 30o bereitgestellt werden. Der untere halbzylinderförmige Gehäuseabschnitt 30u steht auf einem Basisteil 35 und der obere halbzylinderförmige Gehäuseabschnitt 30o kann an einer Deckenhalterung 36 zusätzlich gehalten werden. Das Abgrenzungselement 31 dient dabei gleichzeitig als Trennwand bzw. Wandung W zwischen den beiden Messplätzen M1, M2.

Der hier gezeigte Aufbau hat verglichen mit dem Aufbau der Figur 5 das Ziel, ein homogenes Magnetfeld an den beiden Messplätzen M1, M2 zu erzeugen. Die räumliche Offenheit eines solchen Magnetresonanzscanners 2 ist vergleichbar mit einem C-Magnetsystem, jedoch ist im Unterschied zu einem solchen kein massives Eisenjoch zur Abschirmung bzw. Umlenkung der Magnetfeldlinien vonnöten. Stattdessen wird die Gruppe 41, 42 von Grundfeldmagnet-Segmenten 44 zur Abschirmung und Lenkung des Grundmagnetfeldes B0 verwendet, was das Gewicht gravierend reduziert.

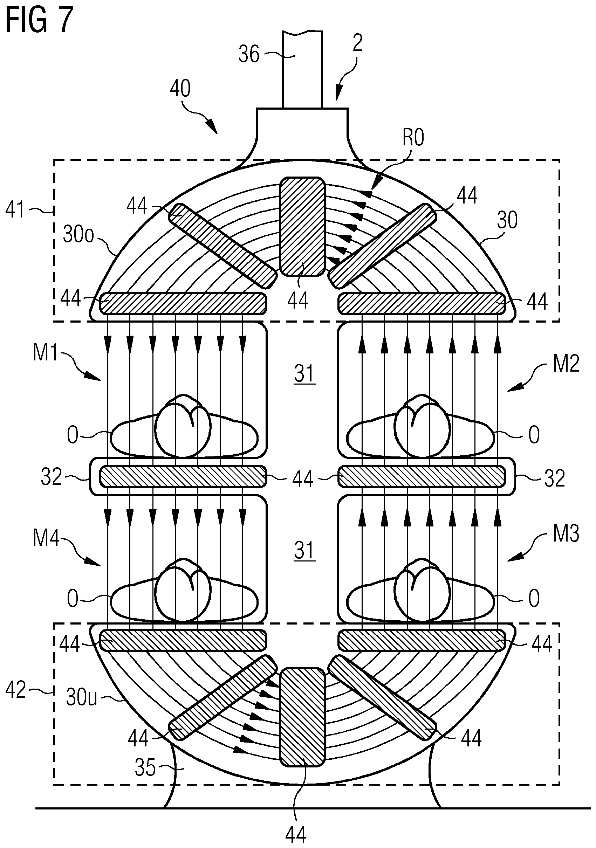

Figur 7 zeigt ein weiteres Ausführungsbeispiel für ein Magnetresonanztomographie-System 1 mit einer weiteren Grundfeldmagnetanordnung 40. Dieses ähnelt sehr dem Aufbau der Figur 6 mit dem Unterschied, dass hier an Stelle von zwei nun vier Messplätze M1, M2, M3, M4 vorhanden sind. Jeweils zwei der Messplätze M1, M2, M3, M4 sind übereinander angeordnet, wobei der jeweils obere vom unteren durch ein Bodenelement 32 des Gehäuses 30 abgetrennt ist. Dieses Bodenelement 32 dient gleichzeitig als Aufnahme eines Grundfeldmagnet-Segmentes 44, welches das Magnetfeld in dem Magnetresonanzscanner 2 homogen durch die Messplätze M1, M2, M3, M4 führt. Die Bodenelemente 32 können beispielsweise an dem als Trennwand bzw. Wandung W zwischen den jeweils benachbarten Messplätzen M1, M2, M3, M4 dienenden Mittelsteg bzw. Abgrenzungselement 31 des Gehäuses 30 verankert sein bzw. sich seitlich aus dieser heraus erstrecken.

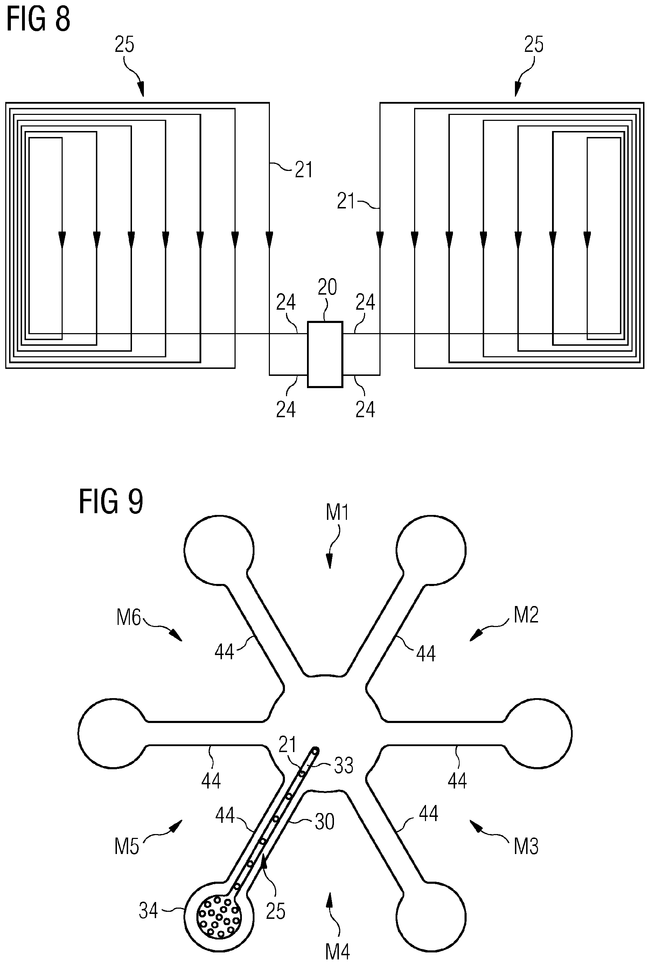

Figur 8 zeigt eine schematische Darstellung von Spulenwicklungen 25 aus Stromleitern 21 zur Kompensation von Inhomogenitäten in einem ringförmigen Magnetfeld Man kann sich vorstellen, dass diese Wicklungen in gegenüber-liegenden Grundfeldmagnet-Segmenten 44 in Figur 3 vorliegen. Wie man deutlich erkennen kann, sind die Spulenwicklungen 25 so gestaltet, dass der Durchmesser einer Wicklung in einer Raumrichtung (nämlich hier der Horizontalen), verglichen mit ihrer benachbarten Wicklung, abnimmt und ihr Mittelpunkt dichter an einem Außenrand des ringförmigen Grundmagnetfelds B0 liegt als der Mittelpunkt der größeren Wicklung. Dadurch wird ein bestimmungsgemäßes Magnetfeld (eines jeweiligen Grundfeldmagnet-Segments 44) erzeugt, das jeweils zu den Außenseiten der Grundfeldmagnet-Segmente 44 hin stärker wird. Dadurch kann - bei geeignetem Aufbau - die im Zusammenhang mit den Figuren 2, 3 und 5 beschriebene, in radialer Richtung verlaufende Inhomogenität des Grundmagnetfelds B0 zumindest teilweise, vorzugsweise vollständig, ausgeglichen werden.

Figur 9 zeigt von oben eine schematische Darstellung einer in Figur 2 dargestellten Ausführungsform mit einer Spulenwicklung 25 nach Figur 8. Hier ist nur ein Grundfeldmagnet-Segment 44 mit einer Detailzeichnung versehen. Es sollte davon ausgegangen werden, dass auch alle anderen Grundfeldmagnet-Segmente 44 solchermaßen aufgebaut sind.

Die Dichte der Stromleiter 21 kann auch mit zunehmenden radialen Abstand zunehmen.

Die Spulenwicklung 25 ist in einem Gehäuseinnenraum 33 des Gehäuses 30 untergebracht. Da die Anzahl der Stromleiter 21 (der Übersichtlichkeit halber ist nur einer bezeichnet) am Rand sehr hoch ist, und ansonsten die Dichte regelmäßig über die Breite eines Grundfeldmagnet-Segments 44 ist, weist diese Grundfeldmagnetanordnung 40 an den äußeren Endabschnitten 34 der Seitenwände der Grundfeldmagnet-Segmente 44 Verdickungen auf, in denen die Stromleiter gebündelt geführt werden.

Anhand der nachfolgenden Figuren werden nun jeweils sehr schematisch verschiedene Ausführungsbeispiele der Erfindung erläutert, bei denen - wie bereits oben erwähnt - "sekundäre" Messplätze Ms oder "Satellitenmessplätze" genutzt werden, die sich in einem nicht oder allenfalls schwach abgeschirmten Streufeld des Grundmagnetfelds der Grundfeldmagnetanordnung befinden. In der Grundfeldmagnetanordnung selbst bzw. im Hauptfeldbereich des Grundmagnetfelds befindet sich jeweils ein "primärer" Messplatz Mp.

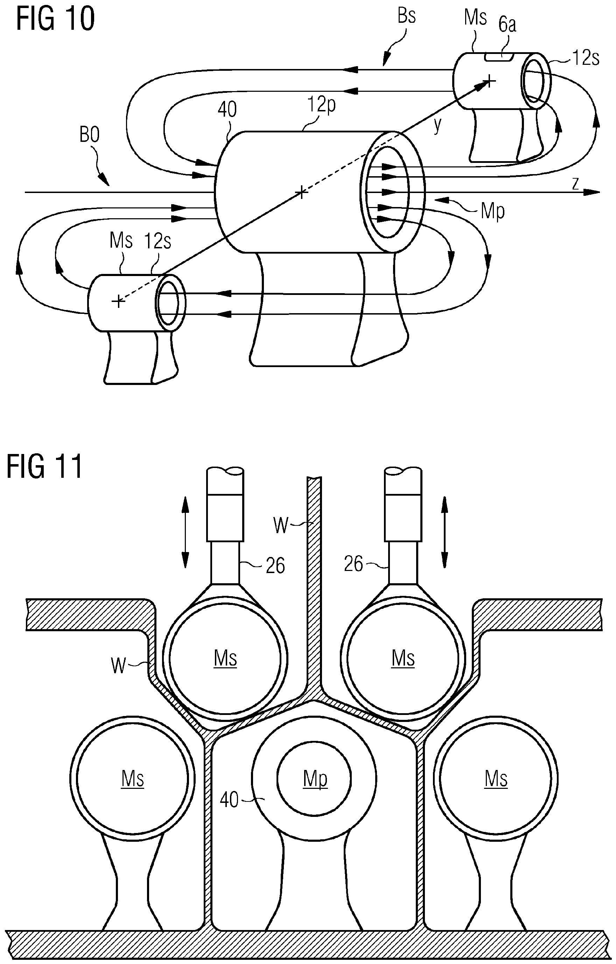

Figur 10 zeigt ein erstes Ausführungsbeispiel für ein solches Magnetresonanztomographie-System 1 gemäß der Erfindung. Der primäre Messplatz Mp liegt hier in einer relativ einfachen Grundfeldmagnetanordnung 40, welche beispielsweise nur einen Grundfeldmagnet in Form eines (insbesondere supraleitender) Solenoid aufweist. Dadurch wird ein sehr homogenes Grundmagnetfeld B0 am primären Messplatz Mp erzeugt, mit dem sehr genaue Messungen mit einer Messeinrichtung 12p möglich sind. Dieser Grundfeldmagnet der Grundfeldmagnetanordnung 40 kann im Prinzip wie der Grundfeldmagnet 4 aus Figur 1 aufgebaut sein, abgesehen von einer geänderten oder zum Teil nicht vorhandenen Magnetfeldschirmung. Es können am primären Messplatz Mp auch alle weiteren Komponenten vorhanden sein, wie sie im Rahmen der Figur 1 erläutert worden sind.

Außerhalb der Grundfeldmagnetanordnung 40 ist in der Figur 10 ein Streumagnetfeld Bs angedeutet, in dem hier zwei sekundäre Messplätze Ms angeordnet sind. An diesen können ebenfalls alle Komponenten vorhanden sein, wie sie im Rahmen der Figur 1 erläutert worden sind, bis auf einen weiteren Grundfeldmagnet 4. Diese "Satellitenmessplätze" Ms weisen insbesondere eine Messeinrichtung 12s auf.

Ein abgeschirmter herkömmlicher Grundfeldmagnet würde bei einem Grundfeld von 3 T in einer Entfernung von 1,5 m immer noch eine Feldstärke von 50 mT im Streufeld Bs haben. Lässt man, wie dies hier bei der Grundfeldmagnetanordnung 40 der Fall ist, die Abschirmung weg, so würde die Stärke im Streumagnetfeld Bs beträchtlich steigen. Beispielsweise liegt hier an den Satellitenmessplätzen Ms noch eine Feldstärke von ca. 0,5 T im Abstand von 1 m bzw. von ca. 100 mT in einem Abstand von 4 m vor.

Die beiden Satellitenmessplätze Ms könnten einen viel größeren Patiententunnel aufweisen (z.B. 80 cm) und kürzer ausgestaltet sein (z.B. 1 m) als der primäre Messplatz Mp. Dies wirkt sich vorteilhaft auf die Vermeidung von klaustrophobischen Ängste aus.

Die Wanddicke der Satellitenmessplätze Ms kann dünner gestaltet werden, z.B. wenn ein unabgeschirmtes Gradientensystem 6 verwendet wird. Dies ist möglich, da man sich vergleichsweise weit weg von der Grundfeldmagnetanordnung 40 befindet.

Um ungewollte Inhomogenitäten im Streufeld Bs zu kompensieren können die sekundären Messplätze Ms ein Shimspulensystem 6a aufweisen (in Figur 10 nur am hinteren Satellitenmessplatz Ms gezeigt). Dies kann ein eigenständiges Spulensystem sein oder im Rahmen eines Gradientensystems 6 (s. Figur 1) realisiert sein.

Figur 11 zeigt ein weiteres Ausführungsbeispiel für ein Magnetresonanztomographie-System 1 gemäß der Erfindung. Neben dem primären Messplatz Mp sind hier vier sekundäre Messplätze Ms so angeordnet, dass das Streumagnetfeld Bs der Grundfeldmagnetanordnung 40 des primären Messplatzes Mp an den sekundären Messplätzen Ms für Messungen verwendet werden kann. Die Messplätze Mp, Ms sind voneinander durch Wandungen W (Seitenwände oder Deckenwände) getrennt, und befinden sich hier alle in unterschiedlichen Räumen, so dass Privatsphäre gewahrt bleibt. Die Wandungen W sind bevorzugt als Faradayscher Käfig ausgeführt, um HF-Übersprecher zu vermeiden. Zusätzlich dienen die Wände auch als eine vorteilhafte akustische Dämmung und optische Abschirmung.

Um die oberen beiden sekundären Messplätze Ms in Bereiche möglichst großer Feldstärke des Streumagnetfeldes Bs zu bringen, lassen sich diese über eine Höhenverstellung 26 für eine Messung in Gruben absenken (Pfeile).

Mit einer solchen Anordnung können bis zu fünf Patienten gleichzeitig untersucht werden, wobei natürlich am primären Messplatz MP die besten Ergebnisse erreicht werden. Wie bereits bei der vorangehend beschriebenen Anordnung benötigt der Grundfeldmagnet 4 keine Abschirmung und die Patiententunnel der sekundären Messplätze Ms kann größer gestaltet werden, also mit einem Messbereich mit größerem Durchmesser und mit kürzerer Längenausdehnung.

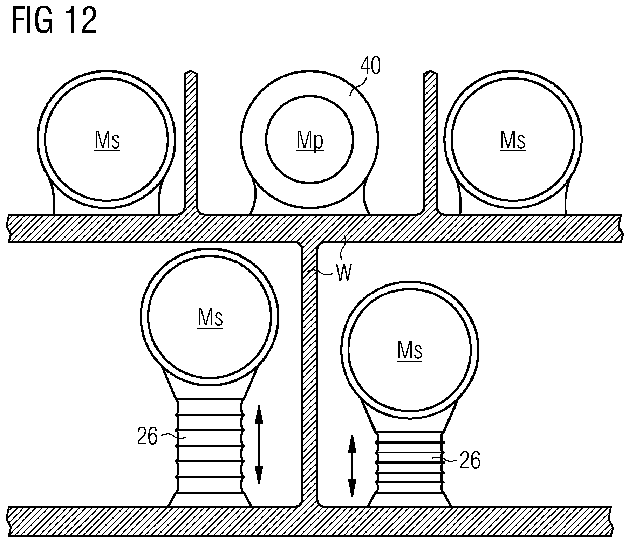

Figur 12 zeigt ein weiteres Ausführungsbeispiel für ein Magnetresonanztomographie-System 1 gemäß der Erfindung. Es ist sehr ähnlich zu Figur 11 mit dem Unterschied, dass sich hier der primäre Messplatz Mp oben befindet und zwei untenliegende sekundäre Messplätze Ms mit einer Höhenverstellung 26 nach oben gehoben werden können.

Eine Höhenverstellung 26 kann beispielsweise durch pneumatische Aktuatoren realisiert werden, die bevorzugt automatisch gesteuert sind.

Eine solche Höhenverstellung 26 erlaubt auch MR-Aufnahmen mit unterschiedlichen Feldintensitäten, insbesondere für ein Verfahren, das als "Field cycling" bekannt ist und eine sehr gute Abbildung der Verteilung eines Kontrastmittels in einem Patienten ermöglicht.

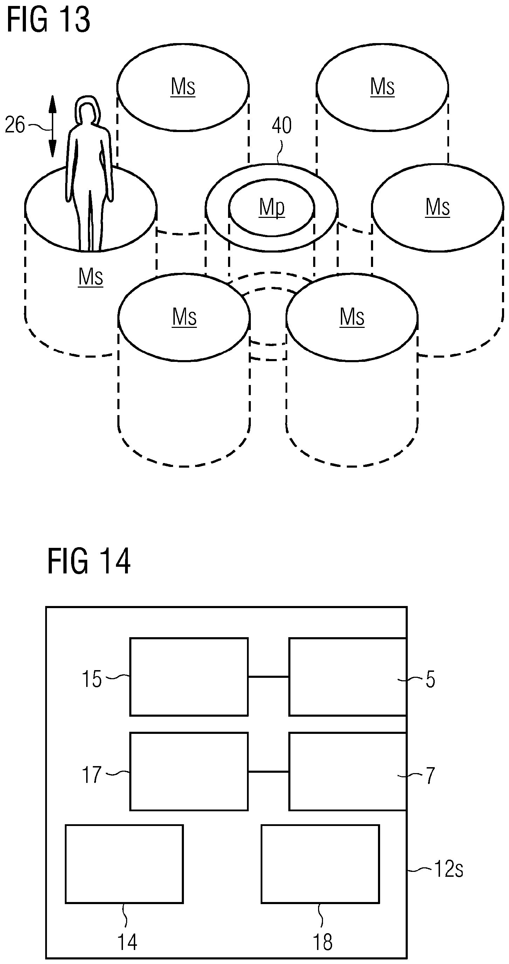

Figur 13 zeigt ein weiteres Ausführungsbeispiel für ein Magnetresonanztomographie-System 1 gemäß der Erfindung mit einem aufrecht stehenden primären Messplatz Mp und sechs sternförmig darum angeordneten ebenfalls aufrecht stehenden sekundären Messplätzen Ms. Mit einem solchen Arrangement könnten z.B. Untersuchungen der Wirbelsäule erfolgen.

Mittels einer Höhenverstellung 26, können Patienten die Messplätze Mp, Ms erreichen, z.B. auf einer Plattform oder mittels einer speziell mit Halterungen versehenen vertikalen "Patientenliege". Nach einer Messung kann der Patient dann wieder nach oben gehoben werden.

Figur 14 zeigt eine grob schematische Block-Darstellung einer Messeinrichtung 12s, wie sie z.B. an einem Satellitenmessplatz Ms angeordnet sein kann. Diese umfasst ein HF-Sendesystem mit einem HF-Sendeantennensystem 5 und einer Hochfrequenzsendeeinrichtung 15, ein HF-Empfangssystem mit einem HF-Empfangsantennensystem 7 und einer Hochfrequenzempfangseinrichtung 17, eine Sequenzsteuereinheit 14 sowie eine Rekonstruktionseinheit 18. Die Messeinrichtung benötigt im Grunde keine Einheit zur Erzeugung eines eigenen Grundmagnetfeldes, z.B. kein U-förmiges Magnetfeld eines Permanentmagneten, da sie ja das Streumagnetfeld einer bestehenden Grundfeldmagnetanordnung verwenden kann. Diese Messeinrichtung könnte auch mobil aufgebaut sein, um temporär verschiedene sekundäre Messplätze mit einer Messeinrichtung auszustatten, Beispielsweise kann die Messeinrichtung als ein tragbares Gerät gestaltet sein, was z.B. bequem in einer Hand Platz findet.

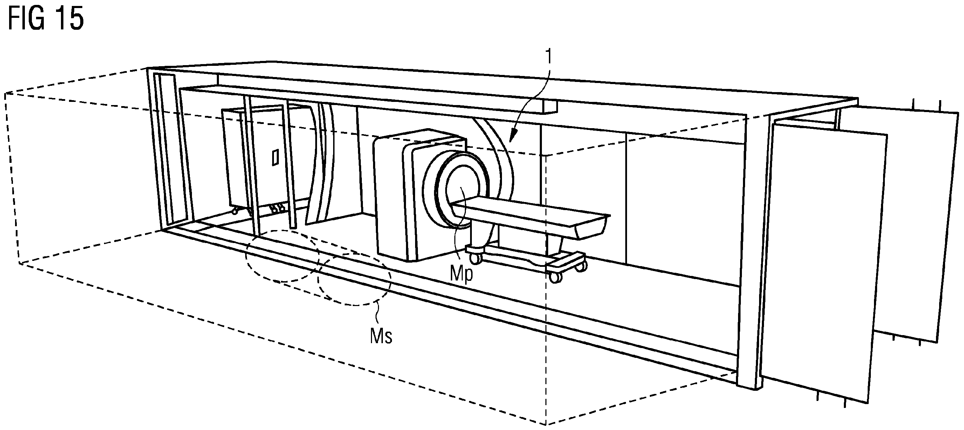

Figur 15 zeigt eine mobile bzw. modulare Ausführungsform eines MR-Systems 1 in einem Container. Neben diesem Container können die sekundären Messplätze Ms liegen, z.B. in weiteren Containern (gestrichelt angedeutet). Die Container sollten keine ferromagnetischen Wände aufweisen, damit das Streumagnetfeld Bs die sekundären Messplätze gut erreichen kann. Mit einem solch modularen Aufbau mittels Containern lassen sich sehr einfach und schnell Behandlungsräume nach den Figuren 11 oder 12 realisieren.

Es wird abschließend noch einmal darauf hingewiesen, dass es sich bei den vorhergehend detailliert beschriebenen Verfahren sowie bei den dargestellten Magnetresonanztomographiesystemen 1 lediglich um Ausführungsbeispiele handelt, welche vom Fachmann in verschiedenster Weise modifiziert werden können, ohne den Bereich der Erfindung, die allein durch die beigefügten Ansprüche definiert ist, zu verlassen. So könnten beispielsweise, wie bereits erwähnt, auch an einem Magnetresonanztomographiesystem, welches mehrere (primäre) Messplätze im Hauptfeldbereich des Grundmagnetfelds bzw. in der Grundfeldmagnetanordnung selber aufweist (wie dies bei der ersten, nicht zur Erfindung gehörenden Hauptvariante der Fall ist), zusätzliche Satellitenmessplätze im Streufeld angeordnet werden (wie dies bei der zweiten Hauptvariante der Erfindung der Fall ist), je nachdem, wie das Grundmagnetfeld aufgebaut und geschirmt bzw. gerade bewusst nicht geschirmt wird. Weiterhin schließt die Verwendung der unbestimmten Artikel "ein" bzw. "eine" nicht aus, dass die betreffenden Merkmale auch mehrfach vorhanden sein können. Ebenso schlie-ßen die Begriffe "Einheit" und "Einrichtung" nicht aus, dass die betreffenden Komponenten aus mehreren zusammenwirkenden Teil-Komponenten bestehen, die gegebenenfalls auch räumlich verteilt sein können.

## Patentansprüche

1. Magnetresonanztomographie-System (1) umfassend eine Grundfeldmagnetanordnung (40) zur Erzeugung eines Grundmagnetfelds (B0) und mehrere räumlich getrennte Messplätze ( Mp, Ms), wobei das Magnetresonanztomographie-System (1) dazu ausgelegt ist, das bestimmungsgemäße Grundmagnetfeld (B0) gemeinsam für die Messplätze ( Mp, Ms) zu nutzen, wobei die mehreren Messplätze aus einem primären Messplatz (Mp), der im Hauptfeld des Grundmagnetfeldes angeordnet ist und mindestens einem sekundären Messplatz (Ms) bestehen, der zur Durchführung einer Messung von Rohdaten im Streufeldbereich des Grundmagnetfelds (B0) angeordnet ist, **dadurch gekennzeichnet, dass** die resultierenden Feldvektoren der bestimmungsgemäßen Magnetfeldverläufe an dem mindestens einen sekundären Messplatz (Ms) um mindestens 30° zum Feldvektor des bestimmungsgemäßen Grundmagnet-Hauptfeldverlaufs an der Position des primären Messplatzes (Mp) geneigt sind.

2. Magnetresonanztomographie-System (1) nach Anspruch 1, welches dazu ausgelegt ist, dass an zumindest zwei der Messplätze ( Mp, Ms) in dem gemeinsamen Grundmagnetfeld (B0) gleichzeitig Magnetresonanztomographieaufnahmen durchgeführt werden können.

3. Magnetresonanztomographie-System (1) nach einem der vorstehenden Ansprüche, umfassend
- zumindest einen primären Messplatz (Mp) innerhalb der Grundfeldmagnetanordnung (40)
und
- zumindest einen sekundären Messplatz (Ms) außerhalb der Grundfeldmagnetanordnung (40),
wobei bevorzugt die resultierenden Feldvektoren der bestimmungsgemäßen Magnetfeldverläufe an dem zumindest einen sekundären Messplatz (MS) entgegengesetzt zum Feldvektor des bestimmungsgemäßen Grundmagnet-Hauptfeldverlaufs an der Position des primären Messplatzes (MP) sind.

4. Magnetresonanztomographie-System (1) nach Anspruch 3, umfassend mindestens zwei an verschiedenen Seiten des primären Messplatzes (Mp) angeordnete sekundäre Messplätze (Ms), welche bevorzugt in einer gemeinsamen Ebene mit dem primären Messplatz (Mp) liegen, und/oder mehrere sternförmig um den primären Messplatz (Mp) herum angeordnete sekundäre Messplätze (Ms).

5. Magnetresonanztomographie-System (1) nach einem der vorangehenden Ansprüche, wobei zumindest ein Messplatz (Mp, Ms), bevorzugt ein sekundärer Messplatz (Ms), eine Höhenverstellung (26) aufweist, mittels derer die Höhe des gesamten Messplatzes (Mp, Ms) und/oder des Objekts verstellt werden kann.

6. Magnetresonanztomographie-System (1) nach einem der vorangehenden Ansprüche 3 bis 5,
wobei zumindest ein sekundärer Messplatz (Ms) in einem anderen Raum angeordnet ist als der primäre Messplatz (Mp) und/oder durch eine Wandung (W) vom primären Messplatz (Mp) getrennt ist,
wobei die Wandung (W) zwischen dem primären Messplatz (Mp) und dem sekundären Messplatz (Ms) bevorzugt paramagnetisch ist und/oder eine akustische und/oder optische Trennung darstellt und/oder die Wandungen um einen Messplatz einen Faradayschen Käfig bilden.

7. Magnetresonanztomographie-System (1) nach einem der vorangehenden Ansprüche 3 bis 6, wobei eine Grundmagnet-Hauptfeldrichtung (R0) des Grundmagnetfelds (B0) im Bereich des primären Messplatzes (Mp) senkrecht zu einer Bodenfläche ausgerichtet ist.

8. Magnetresonanztomographie-System (1) nach einem der vorangehenden Ansprüche, umfassend mindestens zwei, vorzugsweise voneinander unabhängige, Messeinrichtungen (12p, 12s), wobei jede der Messeinrichtungen (12p, 12s) zur Durchführung einer Messung im Rahmen der Magnetresonanztomographie an einem der Messplätze (Mp, Ms) ausgelegt ist, wobei zumindest eine der Messeinrichtungen (12s) mobil ausgestaltet ist.

9. Magnetresonanztomographie-System (1) nach Anspruch 8, wobei eine Messeinrichtung (12s) für einen sekundären Messplatz (Ms) zumindest ein HF-Sendesystem (5, 15) und ein HF-Empfangssystem (7, 17) umfasst und bevorzugt zusätzlich ein Gradientensystem (6) und/oder ein Shimspulensystem.

10. Verfahren zur Messung von Rohdaten für eine Magnetresonanztomographieaufnahme, umfassend die Schritte:
- Positionieren zumindest eines Objekts in einem Messplatz ( Mp, Ms) eines Magnetresonanzto-9, mographie-Systems (1) nach einem der Ansprüche 1 bis
- Erzeugen eines Grundmagnetfelds (B0) mit der Grundfeldmagnetanordnung (40) des Magnetresonanztomographie-Systems (1),
- Messung der Rohdaten.

11. Verfahren nach Anspruch 10, wobei zumindest ein Objekt in einem sekundären Messplatz (Ms) positioniert wird und Rohdaten für Magnetresonanztomographieaufnahmen an diesem sekundären Messplatz (Ms) gemessen werden, wobei bevorzugt an zumindest zwei Messplätzen ( Mp, Ms) gleichzeitig Magnetresonanztomographieaufnahmen durchgeführt werden, bevorzugt wobei nur in einigen Raumrichtungen eine Inhomogenität des Grundmagnetfelds (B0) zur Ortskodierung ausgenutzt wird und in einigen Raumrichtungen zusätzliche Gradientenfelder eingesetzt werden oder dass dies insbesondere von Messplatz ( Mp, Ms) zu Messplatz ( Mp, Ms) unterschiedlich gehandhabt wird.

12. Verfahren nach Anspruch 10 oder 11 wobei eine Inhomogenität des Grundmagnetfelds (B0), insbesondere des Streumagnetfelds (Bs) an einem sekundären Messplatz (Ms), zur Ortskodierung der Rohdaten genutzt wird.

## Claims

1. Magnetic resonance tomography system (1) comprising a basic field magnet arrangement (40) for generating a basic magnetic field (B0) and a plurality of spatially separated measurement stations (Mp, Ms), wherein the magnetic resonance tomography system (1) is configured to use the intended basic magnetic field (B0) collectively for the measurement stations (Mp, Ms), wherein the plurality of measurement stations consists of a primary measurement station (Mp) arranged in the main field of the basic magnetic field and at least one secondary measurement station (Ms) to perform a measurement of raw data in the area of the stray field of the basic magnetic field (B0), **characterised in that** the resulting field vectors of the intended magnetic field profiles at the at least one secondary measuring station (Ms) are inclined by at least 30° to the field vector of the intended basic magnet main field profile at the position of the primary measurement station (Mp).

2. Magnetic resonance tomography system (1) according to claim 1, which is configured to enable magnetic resonance tomography scans to be performed simultaneously at at least two of the measurement stations (Mp, Ms) in the common basic magnetic field (B0).

3. Magnetic resonance tomography system (1) according to one of the preceding claims, comprising
- at least one primary measurement station (Mp) within the basic field magnet arrangement (40)
and
- at least one secondary measurement station (Ms) outside of the basic field magnet arrangement (40), wherein, preferably, the resulting field vectors of the intended magnetic field profiles at the at least one secondary measurement station (MS) are opposite the field vector of the intended basic magnet main field profile at the position of the primary measurement station (MP).

4. Magnetic resonance tomography system (1) according to claim 3, comprising
at least two secondary measurement stations (Ms) which are arranged on different sides of the primary measurement station (Mp) and preferably lie in a common plane with the primary measurement station (Mp),
and/or
a plurality of secondary measurement stations (Ms) arranged in a star shape around the primary measurement station (Mp).

5. Magnetic resonance tomography system (1) according to one of the preceding claims, wherein at least one measurement station (Mp, Ms), preferably a secondary measurement station (Ms), has a height adjustment facility (26) by means of which the height of the entire measurement station (Mp, Ms) and/or of the object can be adjusted.

6. Magnetic resonance tomography system (1) according to one of the preceding claims 3 to 5, wherein at least one secondary measurement station (Ms) is arranged in a different room from the primary measurement station (Mp) and/or is separated from the primary measurement station (Mp) by a wall (W),
wherein the wall (W) between the primary measurement station (Mp) and the secondary measurement station (Ms) is preferably paramagnetic and/or constitutes an acoustic and/or optical separation and/or the walls form a Faraday cage around a measurement station.

7. Magnetic resonance tomography system (1) according to one of the preceding claims 3 to 6, wherein a basic magnet main field direction (R0) of the basic magnetic field (B0) is oriented perpendicularly to a floor surface in the region of the primary measurement station (Mp).

8. Magnetic resonance tomography system (1) according to one of the preceding claims, comprising at least two measuring devices (12p, 12s), preferably independent of one another, wherein each of the measuring devices (12p, 12s) is configured for performing a measurement within the scope of the magnetic resonance tomography scan at one of the measurement stations (Mp, Ms), wherein at least one of the measuring devices (12s) is embodied as mobile.

9. Magnetic resonance tomography system (1) according to claim 8, wherein a measuring device (12s) for a secondary measurement station (Ms) comprises at least one RF transmit system (5, 15) and an RF receive system (7, 17) and preferably in addition a gradient system (6) and/or a shim coil system.

10. Method for measuring raw data for a magnetic resonance tomography scan, comprising the steps:
- positioning at least one object in a measurement station (Mp, Ms) of a magnetic resonance tomography system (1) according to one of claims 1 to 9,
- generating a basic magnetic field (B0) by means of the basic field magnet arrangement (40) of the magnetic resonance tomography system (1),
- measuring the raw data.

11. Method according to claim 10, wherein at least one object is positioned in a secondary measurement station (Ms) and raw data for magnetic resonance tomography scans is measured at said secondary measurement station (Ms), wherein magnetic resonance tomography scans are preferably performed simultaneously at at least two measurement stations (Mp, Ms), preferably wherein an inhomogeneity of the basic magnetic field (B0) is used only in certain spatial directions for spatial encoding and additional gradient fields are applied in certain spatial directions or that this is handled differently from measurement station (Mp, Ms) to measurement station (Mp, Ms) .

12. Method according to claim 10 or 11, wherein an inhomogeneity of the basic magnetic field (B0), in particular of the stray magnetic field (Bs) at a secondary measurement station (Ms), is used for the spatial encoding of the raw data.

## Revendications

1. Système (1) de tomodensitométrie par résonnance magnétique comprenant un agencement (40) de champ magnétique de base pour la production d'un champ (B0) magnétique de base et plusieurs emplacements (Mp, Ms) de mesure séparés dans l'espace, dans lequel le système (1) de tomodensitométrie par résonnance magnétique est conçu pour utiliser le champ (B0) magnétique de base conformément aux prescriptions conjointement pour les emplacements (Mp, Ms) de mesure,
dans lequel les plusieurs emplacements de mesure sont constitués d'un emplacement (Mp) de mesure primaire, qui est disposé dans le champ principal du champ magnétique de base, et d'au moins un emplacement (Ms) de mesure secondaire,
qui
est disposé pour effectuer une mesure de données brutes dans le domaine du champ de dispersion du champ (B0) magnétique de base, **caractérisé en ce que**
les vecteurs de champ résultants des courbes de champ magnétique conformes aux prescriptions sont, en le au moins un emplacement (Ms) de mesure secondaire, inclinés d'au moins 30° par rapport au vecteur de champ de la courbe de champ principal magnétique de base conforme aux prescriptions en la position de l'emplacement (Mp) de mesure primaire.

2. Système (1) de tomodensitométrie par résonnance magnétique suivant la revendication 1, qui est conçu de manière à pouvoir effectuer, en au moins deux des emplacements (Mp, Ms) de mesure dans le champ (B0) magnétique de base commun, en même temps des enregistrements de tomodensitométrie par résonnance magnétique.

3. Système (1) de tomodensitométrie par résonnance magnétique suivant l'une des revendications précédentes, comprenant
- au moins un emplacement (Mp) de mesure primaire dans l'agencement (40) de champ magnétique de base,
et
- au moins un emplacement (Ms) de mesure secondaire à l'extérieur de l'agencement (40) de champ magnétique de base,
dans lequel, de préférence les vecteurs de champ résultants des courbes de champ magnétique conformes aux prescriptions en le au moins un emplacement (MS) de mesure secondaire sont de sens contraire aux vecteurs de champ de la courbe de champ magnétique principal de base conformes aux prescriptions en la position de l'emplacement (MP) de mesure primaire.

4. Système (1) de tomodensitométrie par résonnance magnétique suivant la revendication 3, comprenant
au moins deux emplacements (Ms) de mesure secondaires, qui sont disposés sur des côtés différents de l'emplacement (Mp) de mesure primaire et qui, de préférence, sont dans un plan commun avec l'emplacement (Mp) primaire,
et/ou
plusieurs emplacements (Ms) de mesure secondaires disposés en étoile autour de l'emplacement (Mp) de mesure primaire.

5. Système (1) de tomodensitométrie par résonnance magnétique suivant l'une des revendications précédentes, dans lequel au moins un emplacement (Mp, Ms) de mesure, de préférence un emplacement (Ms) de mesure secondaire, a un réglage (26) de niveau, au moyen duquel le niveau de tout l'emplacement (Mp, Ms) de mesure et/ou de l'objet peut être réglé.

6. Système (1) de tomodensitométrie par résonnance magnétique suivant l'une des revendications 3 à 5 précédentes, dans lequel au moins un emplacement (Ms) de mesure secondaire est disposé dans un espace autre que l'emplacement (Mp) de mesure primaire et/ou est séparé de l'emplacement (Mp) de mesure primaire par une paroi (W),
dans lequel la paroi (W), entre l'emplacement (Mp) de mesure primaire et l'emplacement (Ms) de mesure secondaire est de préférence paramagnétique et/ou représente une séparation acoustique et/ou optique et/ou les parois autour d'un emplacement de mesure forment une cage de faraday.

7. Système (1) de tomodensitométrie par résonnance magnétique suivant l'une des revendications 3 à 6 précédentes, dans lequel une direction (R0) de champ principal du champ (B0) magnétique de base est, dans la partie de l'emplacement (Mp) de mesure primaire, dirigée perpendiculairement à une surface du sol.

8. Système (1) de tomodensitométrie par résonnance magnétique suivant l'une des revendications précédentes, comprenant au moins deux dispositifs (12p, 12s) de mesure, de préférence indépendants l'un de l'autre, dans lequel chacun des dispositifs (12p, 12s) de mesure est conçu pour effectuer une mesure, dans le cadre de la tomodensitométrie par résonnance magnétique, à l'un des emplacements (Mp, Ms) de mesure, dans lequel au moins l'un des dispositifs (12s) de mesure est mobile.

9. Système (1) de tomodensitométrie par résonnance magnétique suivant la revendication 8, dans lequel un dispositif (12s) de mesure pour un emplacement (Ms) de mesure secondaire comprend un système (5, 15) d'émission HF et un système (7, 17) de réception HF et, de préférence, en outre un système (6) de gradients et/ou un système de bobines shim.

10. Procédé de mesure de données brutes pour un enregistrement de tomodensitométrie par résonnance magnétique, comprenant les stades :
- mise en position d'au moins un objet à un emplacement (Mp, Ms) de mesure d'un système (1) de tomodensitométrie par résonnance magnétique suivant l'une des revendications 1 à 9,
- production d'un champ (B0) magnétique de base par l'agencement (40) de champ magnétique de base du système (1) de tomodensitométrie par résonnance magnétique,
- mesure des données brutes.

11. Procédé suivant la revendication 10,
dans lequel on met au moins un objet en position à un emplacement (Ms) de mesure secondaire et on mesure des données brutes pour les enregistrements de tomodensitométrie par résonnance magnétique en cet emplacement (Ms) de mesure secondaire,
dans lequel on effectue, de préférence en au moins deux emplacements (Mp, Ms) de mesure, en même temps des enregistrements de tomodensitométrie par résonnance magnétique,
dans lequel, de préférence on utilise un défaut d'homogénéité du champ (B0) magnétique de base pour un codage spatial et on utilise,
dans certaines directions de l'espace, des champs de gradient supplémentaires ou en ce qu'on fait cette manipulation différemment, en particulier d'un emplacement (Mp, Ms) de mesure à un emplacement (Mp, Ms) de mesure.

12. Procédé suivant la revendication 10 ou 11,
dans lequel on utilise un défaut d'homogénéité du champ (B0) magnétique de base, en particulier du champ (Bs) magnétique de dispersion en un emplacement (Ms) de mesure secondaire, pour le codage spatial des données brutes.
